(19) Europäisches Patentamt European Patent Office Office européen des brevets

(11) **EP 4 230 130 B1**

(12) **EUROPEAN PATENT SPECIFICATION**

(45) Date of publication and mention
of the grant of the patent:
**02.07.2025 Bulletin 2025/27**

(21) Application number: **22158018.6**

(22) Date of filing: **22.02.2022**

(51) International Patent Classification (IPC):
*A61B 5/024* (2006.01)  *A61B 5/022* (2006.01)
*A61B 5/08* (2006.01)  *A61B 5/1455* (2006.01)
*A61B 5/145* (2006.01)  *A61B 5/00* (2006.01)

(52) Cooperative Patent Classification (CPC):
**A61B 5/14532; A61B 5/0022; A61B 5/7405;**
**A61B 5/742; A61B 5/746; A61B 5/747;**
A61B 5/022; A61B 5/024; A61B 5/0816;
A61B 5/1455; A61B 5/6826; A61B 2562/0219

(54) **DEVICE FOR MONITORING AN ANALYTE IN TISSUE OF A HUMAN OR ANIMAL SUBJECT**

VORRICHTUNG ZUR ÜBERWACHUNG EINES ANALYTEN IM GEWEBE EINES MENSCHEN ODER TIERES

DISPOSITIF DE SURVEILLANCE D'UN ANALYTE DANS UN TISSU D'UN SUJET HUMAIN OU ANIMAL

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**23.08.2023 Bulletin 2023/34**

(73) Proprietor: **DiaMonTech AG**
**10245 Berlin (DE)**

(72) Inventors:
• **LUBINSKI, Thorsten**
**10245 Berlin (DE)**
• **SCHRIEK, Uwe**
**10627 Berlin (DE)**

(74) Representative: **Lucke, Andreas**
**Boehmert & Boehmert**
**Anwaltspartnerschaft mbB**
**Pettenkoferstrasse 22**
**80336 München (DE)**

(56) References cited:
CN-A- 108 369 183  US-A1- 2015 150 453
US-A1- 2015 217 002  US-A1- 2020 312 460

**Description**

FIELD OF THE INVENTION

The present invention generally relates to devices for monitoring an analyte in tissue of a human or animal subject. In particular, the present invention relates to devices for non-invasive measurement of analytes in body fluids, such as glucose concentrations in human skin, in particular in the interstitial fluid of human skin.

BACKGROUND OF THE INVENTION

Disclosed is a method of monitoring an analyte in tissue of a human or animal subject. The method comprises a measurement procedure, in which the skin of the subject is brought in contact with a measurement body, which permits heat and/or pressure waves generated by absorption of excitation radiation in the tissue to be transferred to said measurement body. Excitation radiation is irradiated into the tissue to be absorbed by the analyte contained therein, wherein the intensity of said excitation radiation may be time-modulated, and wherein said excitation radiation may comprise radiation of different analyte-characteristic wavelengths that are irradiated one or both of simultaneously and sequentially.

Analyte-characteristic wavelengths as understood herein are wavelengths that allow for determining the presence of an analyte by wavelength-selective absorption, and hence form the basis of the analysis. As such, analyte-characteristic wavelengths may in particular include wavelengths corresponding to absorption maxima of the analyte. Further analyte-characteristic wavelengths are wavelengths corresponding to local minima between two absorption peaks. Namely, the difference between a local absorption minimum and an adjacent peak is a good measure of the concentration of analyte in the tissue. Herein, the term "local minimum" indicates that the absorptivity of the analyte at the given wavelength is smaller than at nearby wavelengths, but is still appreciable, otherwise they would not be characteristic for the analyte. In particular, in preferred embodiments, the absorptivity at these local minima serving as analyte-characteristic wavelengths is more than 5%, preferably more than 10%, more preferably more than 20%, most preferably more than 30% of the highest absorption peak associated with any of the analyte-characteristic wavelengths relied on in the analyte measurement. While wavelengths exactly corresponding with the absorption peaks or local absorption minima are the usually the preferred choices for the analyte-characteristic wavelengths, wavelengths close to the maxima/minima or between maxima and minima may also be used. Accordingly, as understood herein, "analyte-characteristic wavelengths" are also wavelengths where the difference in absorption to that at the closest absorption peak or the closest local absorption minimum is less than 30%, preferably less than 20% of the difference in absorption between the closest absorption peak and closest local absorption minimum. Analyte-characteristic wavelengths may also include wavelengths where the absorption of other substances with which the analyte is mixed in the tissue, is particularly low.

Further, a physical response of the measurement body, or of a component included therein, to heat and/or pressure waves received from said tissue upon absorption of said excitation radiation is detected using a detection device which generates a response signal based on said detected physical response, said response signal being indicative of the degree of absorption of excitation radiation. In the following, the steps of irradiating excitation radiation into the tissue to be absorbed by the analyte contained therein, detecting said physical response of the measurement body and generating said response signal may also be referred to as "performing an analyte measurement".

The present invention is not limited to any specific physical response to heat and/or pressure waves received from said tissue upon absorption of the excitation radiation, nor to any specific way of detecting this physical response in a manner that allows for generating a response signal that is indicative of the degree of absorption of excitation radiation. Various physical responses and corresponding detection methods have been previously proposed for these types of analyte measurement procedure by the present applicant and are briefly summarized below, and each of them may be applied in the present invention.

For example, the detection device may comprise a light source for generating a detection light beam travelling through at least a portion of said measurement body or a component included in said measurement body, and said physical response of the measurement body to heat and/or pressure waves received from said tissue upon absorption of said excitation radiation may be a local change in the refractive index of said measurement body or said component. In this case, the detection device may be configured for detecting one of a change in the light path or a change in the phase of detection light beam due to said change in refractive index of the material of the measurement body or the component included in the same.

For example, in various methods and devices described in detail in two earlier applications of the present applicant, published as Wo 2015/193310 A1 and WO 2017/097824 A1 the measurement body is transparent for said detection light beam, and the detection light beam is directed to be totally or partially reflected at a surface of said measurement body that is in contact with said tissue. In this case, the detection device may comprise a photodetector, in particular a position sensitive photodetector which is capable of detecting a degree of deflection, in particular a deflection angle of said detection light beam due to said local change in refractive index. Accordingly, in this case, the physical response to the heat and/or pressure waves received by the measurement body is a local change in refractive index, and the response signal is the detected degree of deflection which is indeed found to be indicative of the degree of absorption of the excitation radiation.

In alternative variants suggested by the present applicant, as for example disclosed in international application WO 2020/094265 A1 said detection device may comprise an interferometric device allowing for assessing said change in phase of the detection beam and generating a response signal indicative of said change in phase. In this case, the physical response of the measurement body (or a component included therein) to heat and/or pressure waves received from said tissue upon absorption of said excitation radiation is again a local change in index of refraction, while the response signal is in this case an interferometric signal reflecting a change in the phase of the detection beam due to the local change in refractive index.

In yet alternative embodiments, the measurement body or a component in said measurement body may have electrical properties that change in response to a local change in temperature and/or a change in pressure associated therewith, and said detection device comprises electrodes for capturing electrical signals representing said electrical properties. Various possible setups are disclosed in WO 2019/110597 A2. For example, the measurement body may comprise sections having piezoelectric properties, and pressure changes associated with received heat and/or pressure lead to electrical signals that can be recorded with the electrodes. In this case, the change in pressure resembles the physical response of the measurement body or of a component included therein, to heat and/or pressure received from the tissue upon absorption of the excitation radiation, which is detected using the piezoelectric properties of the measurement body and the electrode, and which leads to electrical signals representing the aforementioned response signal that is indicative of the degree of absorption of excitation radiation. In yet further variants, a temperature change due to received heat can be directly measured using very sensitive temperature sensors.

In some examples, the measurement body or a component in said measurement body may comprise a volume in which acoustic waves such as sound waves can propagate, for example a cavity or void, a recess or cut-out and/or an acoustic resonator. The physical response of the measurement body or of said component in the measurement body may thus be a sound wave, e.g. a longitudinal pressure wave, in said volume that is excited by a heat and/or pressure wave received from the tissue upon absorption of the excitation radiation. The detection device may comprise a microphone that is arranged in or in contact with said volume. The microphone may act as a transducer to convert sound waves into electrical signals, wherein the electrical signals may represent the aforementioned response signal that is indicative of the degree of absorption of excitation radiation.

Note that in the following description, the physical response of the measurement body to heat received from the tissue as a result of thermal contact between the measurement body and the tissue is described in detail. However, it is to be understood that in various embodiments of the device of the invention, the tissue is in pressure transmitting contact with the measurement body, and the physical response of the measurement body is a response to pressure waves received from the tissue. Herein, the expression "pressure transmitting contact" shall include all relations that allow for the transfer of pressure waves from the tissue to the measurement body, and in particular, an acoustically coupled relation, where the coupling could be established by a gas, a liquid or a solid body. All detailed explanations given in connection with the thermal contact and physical response to heat received by the measurement body from the tissue shall be understood in combination with scenarios including pressure transmitting contact and physical response to pressure waves, where applicable, without explicit mention.

Note that the term "analyte measurement procedure" or "analyte measurement" indicates that this measurement procedure is based on response signals obtained with excitation radiation at analyte-characteristic wavelengths.

The method may further comprise an analyzing step, in which said analyzing is carried out based, at least in part, on said response signal. Since in this case, the response signal is indicative of the degree of absorption of excitation radiation comprising "analyte-characteristic wavelengths", the response signal is directly related to the concentration of the analyte in the tissue. Accordingly, the analyzing step is based at least in part on a measure of the concentration of the analyte in the tissue, and in some non-limiting applications, it may actually amount to determining this concentration.

For example, the above method has been employed by the applicant in devices for non-invasive measuring of a glucose level of a user. In this specific application, the "analyte" is formed by glucose, and the "tissue" is the skin of the user. It has been previously demonstrated that this method allows for very precise measurement of glucose concentration in the interstitial fluid within the skin of a person, which is found to be directly related with and hence representative of the glucose content of the patient's blood. Shown in Fig. 4 of the present application is the result of a Clark's error grid analysis taken from WO 2017/097824 A1, demonstrating that the above analysis method allows for predicting the actual glucose concentration of a person very precisely.

Another example of a device for performing such analyte measurements is known from CN 108 369 183 A. The device disclosed therein may comprise a housing with a belt for attaching the housing to the body of a person and a display for displaying measurement or analysis results such as a glucose concentration.

The above method may be implemented in stationary apparatuses, which may for example be used in a clinical setting or in a patient's home for monitoring an analyte level such as a glucose level of the user or patient (subject). While this may provide valuable information on a state of the user in everyday situations, e.g. by monitoring the glucose level at regular intervals, what is lacking are devices and methods that provide information pertaining to the state of the patient in emergency situations, in particular when the user is unconscious or otherwise unresponsive. As a results of this, valuable time may be lost by first responders. Moreover, inappropriate or wrong measures may be taken in particular by inexperienced first responders such as random bystanders, who might for example assume that an unresponsive persons is drunk rather than for example a hypoglycemic person with diabetes.

SUMMARY OF THE INVENTION

It is thus an object of the present invention to provide means for facilitating first aid for patients with medical conditions such as diabetes in emergency situations.

This object is met by a device for monitoring an analyte in tissue of a human or animal subject according to claim 1. Examples of the present invention are detailed in the dependent claims.

The device for monitoring an analyte in tissue of a human or animal subject according to the invention comprises a measurement body having a contact surface suitable to be brought in contact with a skin of the subject, said contact permitting heat and/or pressure waves generated by absorption of excitation radiation in the tissue to be transferred to said measurement body. The device further comprises an excitation radiation source configured for irradiating excitation radiation at a plurality of wavelengths into the tissue to be absorbed by the analyte contained therein. The device also comprises a detection device for detecting a physical response of the measurement body or of a component included therein to a heat and/or pressure wave received from the tissue upon absorption of the excitation radiation and for generating a response signal based on said detected physical response, said response signal being indicative of a degree of absorption of excitation radiation. The device further comprises a controller to control the excitation radiation source to irradiate excitation radiation into the tissue to be absorbed by the analyte contained therein and to control the detection device to detect said physical response and to generate a response signal indicative of the degree of absorption of said excitation radiation. The device also comprises a holding portion for securing the device to a body part, in particular a limb of the subject, and a communication unit. The communication unit is configured to provide monitoring information pertaining to the degree of absorption of said excitation radiation and/or a first aid instruction based on the degree of absorption of said excitation radiation by acoustic, optical, and/or wireless communication means. The controller is configured to determine a state of consciousness of the subject that characterizes whether the subject is conscious and/or responsive, wherein the state of consciousness is determined based on said response signal and/or based on a sensor signal from one or more auxiliary sensors configured to measure a parameter indicative of whether the subject is conscious and/or responsive. The controller is further configured to, if the state of consciousness indicates that the subject is unconscious and/or unresponsive, provide monitoring information pertaining to the degree of absorption of said excitation radiation and/or a first aid instruction based on the degree of absorption of said excitation radiation via the communication unit by one or more of said acoustical, optical, and/or wireless communication means.

The device may for example be a wearable device, e.g. a wearable device that is configured to be worn by the subject by securing or fastening the wearable device to said body part with the holding portion. In other words, physical dimensions and the weight of the device may be such that the device can be carried by the subject, e.g. such that the device can be worn by the subject over extended periods of time (e.g. for more than one hour) or permanently. In some embodiments, the device can for example be a smartwatch, a fitness tracker or a chest-strap monitor. The wearable device may be configured for use on human subjects (e.g. a user or a patient), but may also be adapted for use on animal subjects, e.g. by adjusting the physical dimensions and/or the shape of the wearable device accordingly, for example so as to fit on the respective body part. The animal subjects may be mammals, in particular larger mammals (e.g. mammals with a body size on the same order of magnitude as humans) such as cats, dogs, pigs and cows.

The analyte is not particularly limited and may be any substance that absorbs excitation radiation at a plurality of analyte-characteristic wavelengths. Preferably, the analyte is a physiologically relevant substance that is present in tissue of humans and/or animals. The analyte may in particular be glucose.

The tissue may be any tissue in the human or animal body that can be irradiated by excitation radiation and from which heat and/or pressure waves may be transferred to the measurement body. The tissue may for example be sufficiently close to the skin of the subject for the tissue to be reached by excitation radiation irradiated through the skin of the subject and/or for heat and/or pressure waves generated by absorption of the excitation radiation in the tissue to propagate to the surface of the skin. The tissue may in particular be the skin of the subject. In one example, the analyte is glucose, in particular glucose present in an interstitial fluid of the skin, i.e. in a fluid present in a space between cells within the skin. While in the following explanations and in the description of the specific embodiments reference may be made to this embodiment, it is to be understood that the invention is not limited to this, and that it can be applied to other types of tissue and/or analytes.

The measurement body may be any body that (or a component thereof) exhibits a physical response to heat and/or pressure waves that can be detected with a detection device, e.g. as described above. The measurement body may be a solid body, in particular a transparent solid body. In some embodiments, the body may also comprise a fluid, which may e.g. be contained in a cavity or void within the measurement body. The contact surface may be a surface of the measurement body that is exposed from the device, i.e. not covered by any other part of the device, such that it can be brought in contact with the skin of the subject. The contact surface may be a simply connected surface (i.e. without holes), but may also for example be a surface extending around a recess or cut-out in the measurement body (e.g. an edge or rim of the recess). The contact between the measurement body and the skin may be a thermal contact that permits the transfer of heat waves to the measurement body, e.g. through the contact surface, and/or may be a pressure transmitting contact that permits the transfer of pressure waves to the measurement body, e.g. through the contact surface and/or through an interface or plane surrounded by the contact surface (e.g. a "surface" or opening of a recess in the measurement body that is surrounded by the contact surface).

The excitation radiation source is configured to generate excitation radiation at a plurality of wavelengths ("excitation wavelengths"), in particular at a plurality of analyte-characteristic wavelengths. Said plurality of wavelengths or analyte-characteristic wavelengths may for example comprise between 2 and 50 wavelengths, in some examples between 5 and 20 wavelengths. The excitation radiation source may for example be a light source such as a laser that is configured to generate excitation radiation in the optical spectrum, for example in the infrared spectrum, in particular in the near-infrared and/or mid-infrared spectrum. For irradiating the excitation radiation generated by the excitation radiation source into the tissue, e.g. through the contact surface and/or through the measurement body, the device may comprise one or more optical elements such as waveguides, lenses and/or mirrors.

In some embodiments, said excitation radiation is generated using an array of lasers, in particular semiconductor lasers, further in particular quantum cascade lasers, each having a dedicated wavelength. Each of the lasers may have its own modulation device, or they may have a common modulation device and they may be controlled by a common or by individual control units. The lasers of an array may be optically aligned in a way that allows for using a common optical path for the excitation beam and they may for example radiate into the tissue through a common light waveguide. The laser array may be combined on a single semiconductor chip.

In some embodiments, said excitation radiation is generated using at least one tunable laser, in particular at least one tunable quantum cascade laser.

In a preferred embodiment, some or all of said excitation wavelengths are in a range of 5 $\mu$m to 13 $\mu$m, preferably 8 $\mu$m to 11 $\mu$m. In alternative embodiments, some or all of said excitation wavelengths are in a range of 3 $\mu$m to 5 $\mu$m. This wavelength range is for example useful for detecting absorption of $CH_2$ and $CH_3$ vibrations in fatty acids.

The detection device may be any device that is configured to detect the physical response of the measurement body or of the component therein to heat and/or pressure waves and to generate a corresponding response signal, e.g. as detailed above. The detection device may comprise one or more detection elements such as a photodetector, an electrode, a temperature sensor and/or a microphone for detecting the physical response. The detection device may further comprise one or more probing sources such as a light source and/or a current or voltage source for generating a probing excitation such as a light beam and a current or voltage, respectively, that is to be detected by the one or more detection elements, e.g. to detect an effect of a heat and/or pressure wave on the probing excitation such as a deflection of the light beam. The response signal generated by the detection device may allow for quantifying the physical response of the measurement body and thereby the degree of absorption of excitation radiation in the tissue.

In some embodiments, said detection device comprises a light source for generating a detection light beam travelling through at least a portion of said measurement body or a component included in said measurement body, said physical response of the measurement body to heat received from said tissue upon absorption of said excitation radiation is a local change in the refractive index of said measurement body or said component, and said detection device is configured for detecting one of a change in the light path or a change in the phase of detection beam due to said change in refractive index and for generating a response signal indicative of said change in light path or phase of the detection beam.

In some embodiments, said measurement body is transparent for said detection light beam, said detection light beam is directed to be totally or partially reflected at a surface of said measurement body that is in contact with said skin, and wherein said detection device comprises a photodetector, in particular a position sensitive photodetector, capable of detecting a degree, in particular angle of deflection of said detection light beam due to said local change in refractive index.

In some embodiments, said detection device comprises an interferometric device allowing for assessing said change in phase of the detection beam and generating a response signal indicative of said change in phase.

In some embodiments, said measurement body or a component in said measurement body has electrical properties that change in response to a local change in temperature or a change in pressure associated therewith, and wherein said detection device comprises electrodes for capturing electrical signals representing said electrical properties.

The controller is configured to perform analyte measurements for detecting the analyte in said tissue, i.e. to control the excitation radiation source to irradiate excitation radiation, in particular excitation radiation at a plurality of analyte-characteristic wavelengths, into the tissue to be absorbed by the analyte contained therein and to control the detection device to detect said physical response and to generate a response signal indicative of the degree of absorption of said excitation radiation. The controller may be configured to control the excitation radiation source such that the intensity of said excitation radiation is time-modulated (e.g. pulsed) and/or such that different analyte-characteristic wavelengths that are irradiated one or both of simultaneously and sequentially.

The controller may be embodied in hardware, software or both. In particular, the controller may comprise one or more computers, processors, microcontrollers, FPGAs ASICs and corresponding computer programs which when executed on the corresponding hardware provide the control functions described herein. In some examples, the controller may be a distributed system, for example comprising several control units that are in data communication with each other, of which some may be provided in a housing of the device, and others remote from the housing, but it can also be formed by a single control unit. To control components of the device such as the excitation radiation source and the detection device, the controller may be configured to generate digital and/or analog control signals for the respective component. In a preferred embodiment of the device, said controller is configured to control components of the device for carrying out a method for monitoring an analyte in tissue of a human or animal subject according to any one of the embodiments described herein at least in part, in one example in its entirety (i.e. to execute all steps of said method).

The controller may further be configured to perform an analyzing step, for example as described above. The controller may in particular be configured to determine a measure of the concentration of the analyte in the tissue, e.g. the concentration itself or a quantity that has a known functional relation to the concentration (for example is proportional to the concentration), based on the response signal.

When the excitation radiation is radiated into the tissue, part of it will be absorbed along its light path, e.g. at different depths below the surface (i.e. the skin), for example by the analyte contained within the tissue. In response to the absorption, a heat signal is generated, which diffuses back to the surface/skin and is received by the measurement body. When the intensity of the excitation radiation is modulated, the tissue is heated by absorption in a time-dependent manner, leading to a temperature field that varies as a function of space and time and that is often referred to as a thermal wave. The term thermal "wave" is somewhat misleading, since the travel of heat through the material is not governed by a wave equation, but by a diffusion equation instead. This also means that the largest depth of the modulated absorption underneath the surface that can be detected by heat received by the measurement body at the surface is limited approximately by the so-called diffusion length $\mu_t$, which depends on the density $\rho$, the specific heat capacity $C_p$, and the thermal conductivity $k_t$ of the tissue as well as on the modulation frequency f of the excitation radiation:

$$\mu t(f) = \sqrt{\frac{k_t}{\rho \cdot C_p \cdot 2 f}}$$

Since the interstitial fluid is only found in the tissue at a certain range below the surface of the skin, in order to generate response signals indicative of absorption in the interstitial fluid, the modulation frequency of the excitation radiation has to be indeed "sufficiently low" such that the diffusion length $\mu_t$ is long enough to cover the distance between regions with interstitial fluid and the skin surface. However, as explained above, the diffusion length depends on the physical properties of the tissue, namely density $\rho$, the specific heat capacity $C_p$, and the thermal conductivity $k_t$, and the present applicant noticed that these properties will not only vary from person to person, but may also vary for the same person with time, depending for example on whether the skin is dry or moist, whether the skilled person has used hand moisture lotion, or the like. In other words, the thermal diffusion length as a function of frequency is a material/tissue status that for best results should be assessed at or close to the time of the analyte measurement procedure in a material/tissue status analyzing procedure, e.g. as described in WO 2021/233559 A1. Thereby, a modulation frequency may be determined that is "sufficiently low" such that the response signal reflects at least in part absorption of excitation radiation within the interstitial fluid.

Moreover, the depth below the surface at which the interstitial fluid resides is found to be dependent on the thickness of the stratum corneum, which again does not only vary from person to person, but also varies for the same person over time and also over the location of the skin surface where the measurement takes place. For example, if a person has recently done physical work with his or her hands, or has practiced a string instrument, the stratum corneum may be thicker than usual, meaning that the absorption has to be effected in deeper layers below the surface of the skin in order to cover the interstitial fluid. Again, the thickness of the stratum corneum is a "material/tissue status" that can be assessed in said material/tissue status analyzing procedure.

However, even if the thermal diffusion length is sufficiently long that the response signal reflects at least in part absorption of excitation radiation within the interstitial fluid, the response signal will also represent any absorption in upper layers of the skin, in particular the stratum corneum, and hence include a contribution that is not related to the glucose within the interstitial fluid. Indeed, this contribution from upper layers of the skin, in particular the stratum corneum, will be typically overrepresented in the response signal, since the intensity of the excitation radiation in the upper layers is higher than deeper into the skin, and since the absorption heat needs to travel only a shorter distance from the upper layers to reach the measurement body. In order to estimate this contribution of higher layers of the skin to the (first) response signal, at least one additional second response signal may be recorded in response to excitation radiation with a second modulation frequency, which is higher than the other (first) modulation frequency, and hence leads to a shorter diffusion length and therefore represents absorption predominantly in the upper layers of the skin above the region where the interstitial fluid resides. Then, response signals corresponding to said modulation frequencies, or quantities derived therefrom, are mathematically combined to yield information more specifically indicative of the absorption in the interstitial fluid. There are different ways of mathematical combining these response signals, and this aspect of the invention is not limited to any single one of them. For example, the second response signal can be multiplied with a normalizing factor that is obtained e.g. based on reference measurements with excitation wavelength(s) for which glucose absorption is negligible, and then the product may be subtracted from the first response signal.

The holding portion is configured to secure the device to a body part of the subject such as a limb. Preferably, said body part is an upper limb of the subject such as an arm or a part thereof, in particular an upper arm and/or a wrist. The holding portion may be configured to be arranged around said body part such that the holding portion extends around the body part along a circumferential direction (e.g. substantially parallel to a surface of said body part). In other words, when arranged around said body part, the holding portion may extend around a circumference of the body part. When arranged around said body part, the device and/or the holding portion may extend around the body part completely (by 360°, e.g. to form a closed loop enclosing said body part such as a closed wristband) or partially (by less than 360°, e.g. to form a bracket- or clip-like structure such as an open wristband). The device and/or the holding portion may for example extend around the body part by between 180° and 360°, preferably between 270° and 360°. In some examples, the holding portion may have a substantially circular or substantially elliptical shape. In some embodiments, the holding portion or a part thereof may be attached to a main housing of the device, wherein the main housing may e.g. contain or hold some or all of the measurement body, the excitation radiation source, the detection device and the controller. A first end of the holding portion may for example be attached to a first side of the main housing and a second end of the holding portion may be attached to a second side of the main housing opposite to the first side, e.g. so as to form a closed loop. The main housing may be part of the holding portion, e.g. to close the loop, and in some examples may be formed integrally with the holding portion or a part thereof.

In some embodiments, the device may be configured to temporarily increase a contact pressure between the measurement body and the skin of the subject, e.g. to establish and maintain close contact for performing an analyte measurement. The device may for example be configured to temporarily increase the contact pressure to between 0.1 N/cm$^2$ and 100 N/cm$^2$, preferably to between 1 N/cm$^2$ and 10 N/cm$^2$. To temporarily increase the contact pressure, the device may comprise actuated means that are configured to reversibly perform one or more of (1) reducing an inner diameter of the device, wherein the inner diameter of the device is a distance between two opposing portions of the device being arranged on opposite sides of the body part when the device is secured to the body part; (2) reducing an inner circumference of the device, wherein the inner circumference of the device is a length of an inner surface of the device facing the body part when the device is secured to the body part; and (3) moving the measurement body or a part thereof radially inward towards the body part (e.g. in a direction that is perpendicular or substantially perpendicular to the surface (e.g. the skin) of said body part). Loosely speaking, the actuated means may be configured to tighten the device (e.g. the holding portion) and/or to move the measurement body closer to the body part in order to ensure a tight fit and close contact between the measurement body and the skin. Preferably, the controller is configured to control the actuated means to temporarily increase the contact pressure.

Said actuated means for temporarily increasing the contact pressure may for example comprise one or more actuators, wherein the one or more actuators may e.g. include one or more of an electric actuator (e.g. an actuator comprising an electric motor), a magnetic actuator (e.g. an actuator configured to generate or switch a magnetic field to move a magnetic object or an electromagnet), a piezoelement (e.g. an element comprising or consisting of a piezoelectric material) and a fluid pump.

Additionally or alternatively, said actuated means for temporarily increasing the contact pressure may comprise one or more deformable members configured to be deformed, extended and/or retracted reversibly. The one or more deformable members may for example comprise one or more of a bimetallic element (e.g. an element such as a strip or coil comprising two or more materials, in particular metals, having different coefficients of thermal expansion), a shape-memory alloy structure (e.g. a structure or element that has a first shape (e.g. straight) at a first temperature and a second shape (e.g. bent) at a second temperature different from the first temperature), a bistable spring assembly (e.g. an assembly comprising one or more springs that can be compressed/stretched between two stable configurations, which may for example be defined by the arrangement of the springs and/or respective mechanical stops or detents), a pneumatically and/or hydraulically extendable member (e.g. a hydraulic or pneumatic cylinder or a pneumatically or hydraulically movable shaft or piston) and an inflatable body (e.g. a body enclosing a volume that is configured to receive a fluid such as air to be expanded from a compressed state to an expanded state).

In some embodiments, the measurement body and the excitation radiation source may be arranged in different parts of the device, in particular in different parts or segments of the holding portion. In other words, the measurement body and the excitation radiation source may be positioned spaced apart from each other, e.g. such that the measurement body is rotated by an angle between 30° and 330°, in some examples between 90° and 270°, in one example between 150° and 210°, along the circumferential direction around the body part with respect to the excitation radiation source. This may for example allow for arranging the measurement body such that, when the device is secured to a wrist or lower arm of a human subject, the measurement body is arranged on an inside of the wrist or lower arm (i.e. facing towards the torso of the subject) when a main housing of the device is arranged on an outside of the wrist or lower arm (i.e. facing away from the torso). In other words, the main housing (which may e.g. house the controller and a display) and the measurement body may be arranged on opposite sides of the device. The excitation radiation source may for example be arranged in or on the main housing or in or on a part or segment of the device and/or of the holding portion adjacent to the main housing. In another example, the measurement body is arranged in or on the main housing and the excitation radiation source is arranged in or on another part of the device and/or of the holding portion, which may e.g. be adjacent to the main housing.

The device may further comprise a waveguide configured to guide excitation radiation from the part of the device in which the excitation radiation source is arranged to the part of the device in which the measurement body is arranged. The waveguide may for example be an optical fiber, e.g. a multimode optical fiber. The waveguide may extend along or through a part of the holding portion. In one example, the waveguide extends from the excitation radiation source to the measurement body, e.g. to guide excitation radiation from an output of the excitation radiation source to the measurement body and to couple the excitation radiation into the measurement body.

The communication unit may be configured to provide monitoring information pertaining to the degree of absorption of said excitation radiation by acoustic, optical, and/or wireless communication means. Said monitoring information may in particular pertain to or comprise a measure for the concentration of the analyte, for example pertain to or comprise the concentration of the analyte itself, e.g. as detailed below. The monitoring information may for example be based on the most recent analyte measurement or on a plurality of measurements. The monitoring information may for example comprise a time trace based on the most recent analyte measurements, e.g. a time trace of the concentration of the analyte.

Additionally or alternatively, the communication unit may also be configured to provide one or more first aid instructions, wherein the first aid instructions are based on the degree of absorption of said excitation radiation at least in part. The first aid instructions may for example be instructions for actions or measures to be taken, e.g. by first responders, that are associated with certain values or ranges of the concentration of the analyte. For example, the analyte may be glucose and the first aid instruction may either be to give food (or water) to the user or to not give food (or water) to the user depending on the glucose level, e.g. depending on whether the patient is hypoglycemic or hyperglycemic.

Said communication means may for example comprise one or more of a loudspeaker, a display, an optical indicator (e.g. one or more light sources such as light-emitting diodes, LED) and a wireless communication module. The wireless communication module may for example be configured to exchange data with an external device such as a portable computing device (e.g. a smartphone, a smartwatch or a tablet) or a telephone (e.g. a telephone of an emergency call center) via one or more wireless communication methods known in the art. Said wireless communication methods may for example comprise one or more of a cellular network (e.g. a 4G and/or 5G cellular network), a wireless network (e.g. Wi-Fi), a short-range wireless technology such as Bluetooth and satellite communication channels. The device may comprise some or all of said communication means. Additionally or alternatively, some or all of said communication means may be comprised in an external device at least in part.

The controller is configured to determine the state of consciousness of the subject based on the response signal (i.e. based on an analyte measurement) and/or based on a sensor signal from one or more auxiliary sensors, e.g. as detailed below. The controller is further configured to provide monitoring information pertaining to the degree of absorption of said excitation radiation and/or one or more first aid instructions based on the degree of absorption of said excitation radiation via the communication unit by one or more of said acoustical, optical, and/or wireless communication means in response to the state of consciousness indicating that the subject is unconscious and/or unresponsive.

For example, the controller may be configured to show the concentration of the analyte on a display of the device. Additionally or alternatively, the controller may activate an optical indicator such as an LED, which may e.g. indicate that the concentration of the analyte is below a threshold (e.g. a pre-defined threshold), and/or a plurality of optical indicators such as a row or array of LEDs, wherein a number of activated optical indicators may e.g. indicate the concentration of the analyte. The one or more optical indicators may for example be arranged next to corresponding labels on the device to indicate or explain their meaning.

Preferably, the controller is configured to provide the monitoring information by acoustic means, which may be easier to notice, recognize or understand for first responders in case of emergency. For example, the controller may be configured to announce the concentration of the analyte via a loudspeaker, e.g. to read the concentration of the analyte aloud, and/or to announce that the concentration of the analyte is below a threshold (e.g. to announce that the subject is hypoglycemic). Additionally or alternatively, the controller may also be configured to provide the monitoring information to an emergency call center, a public safety answering point or professional first responders by wireless communication means, e.g. as part of an emergency call.

Additionally or alternatively, the controller may be configured to provide one or more first aid instructions that are based on the degree of absorption of the excitation radiation, for example on the concentration of the analyte, e.g. as detailed above. The controller may in particular be configured to provide said first aid instructions by acoustical means, e.g. by acoustic playback on a loudspeaker. The controller may be configured to determine the first aid instructions based on the degree of absorption of the excitation radiation, e.g. using a corresponding look-up table. For determining the first aid instructions, the controller may further take into account medical information pertaining to a general state of health of the subject, e.g. whether the subject as a certain medical condition such as diabetes.

Recent advances in the miniaturization of components such as lasers for generating excitation radiation at a plurality of analyte-characteristic wavelengths enable the implementation of a method for detecting an analyte as set forth above in ever small devices. In particular, the method can be implemented in devices that are small enough to by carried around by a user over extended periods of time or even permanently, for example in wearable devices such as smartwatches, fitness trackers or chest-strap monitors (e.g. heart-rate monitors). By determining the state of consciousness and providing monitoring information pertaining to the degree of absorption of the excitation radiation (e.g. the most recent glucose level) and/or first aid instructions based on the degree of absorption of said excitation radiation (e.g. instructions that are specifically tailored to a particular range of values of the analyte concentration/glucose level) by acoustic, optical and/or wireless communication means in case the subject becomes unconscious or unresponsive, valuable information may be provided to first responders in emergency situations. This may not only save time, but may also ensure that the first responders take appropriate actions, for example to either provide or not provide food to a patient with diabetes.

In a preferred embodiment, the controller is configured to determine a concentration of the analyte in the tissue based on the response signal generated by the detection device, e.g. using a corresponding calibration curve and/or a corresponding classifier (e.g. a neural-network based classifier). The monitoring information may quantify the determined concentration of the analyte (e.g. comprise the value of the concentration or of a measure for the concentration) and/or may indicate whether the determined concentration is below or above one or more thresholds (e.g. pre-defined thresholds). If the analyte is glucose, the monitoring information may for example indicate whether (and optionally to what extent) the subject is hypoglycemic (i.e. has low blood sugar, e.g. below 70 mg/dL) and/or hyperglycemic (i.e. has high blood sugar, i.e. above 200 mg/dL). Additionally or alternatively, the controller may be configured to provide different first aid instructions depending on whether the determined concentration is below or above one or more thresholds (e.g. pre-defined thresholds). In one example, the controller is configured to provide a first first aid instruction (e.g. to not give food to the subject) if a concentration of the analyte is above a threshold and to provide a second first aid instruction (e.g. to give food to the subject) if the concentration of the analyte is below the threshold.

In some embodiments, the controller may further be configured to provide medical information pertaining to a general state of health of the subject, in particular to one or more medical conditions of the subject, via the communication unit if the state of consciousness indicates that the subject is unconscious and/or unresponsive. The medical information may for example indicate whether the subject has a certain medical condition (e.g. diabetes or an allergy). The medical information may further comprise other information that may be useful for first responders such as for example an age of the user and/or a blood type of the user. The controller may for example be configured to control the communication unit to display on a display and/or to announce via a loudspeaker that the subject has diabetes.

The controller may be configured to determine the state of consciousness based on the sensor signal from the one or more auxiliary sensors configured to measure a parameter indicative of whether the subject is conscious and/or responsive. In the context of this disclosure, an auxiliary sensor may for example be a sensor that is per se unrelated to the measurement apparatus for measuring the analyte absorption. In other words, an auxiliary sensor may be a sensor that is (primarily) used for other purposes than for detecting or monitoring the analyte. The controller may be configured to determine a value and/or a time trace of the respective parameter from or based on the corresponding sensor signal and/or to obtain said value and/or said time trace from the respective auxiliary sensor. To determine the state of consciousness, the controller may be configured to compare said parameters and/or a rate of change of said parameters to one or more thresholds.

An auxiliary sensor may for example be a pulse sensor, in particular an optical pulse sensor, configured to determine a pulse of the subject or an oxygen saturation sensor, in particular an optical oxygen saturation sensor, configured to determine an oxygen saturation level of blood of the subject. The pulse and the oxygen saturation are examples of parameters that are indicative of whether the subject is conscious and/or responsive. The controller may for example be configured to determine the state of consciousness by determining one or more of whether the pulse is below a threshold (e.g. a pre-defined threshold), whether the pulse is above a threshold (e.g. a pre-defined threshold) and whether a time trace of the pulse exhibits one or more characteristic features that may be associated with unconsciousness (e.g. a sudden change), for example a rate of change of the pulse being above a threshold (e.g. a pre-defined threshold). The subject may for example be assumed to be unconscious and/or unresponsive if the pulse is below the threshold for more than a pre-defined amount of time. A similar analysis may additionally or alternatively be performed based on the oxygen saturation.

Other examples for auxiliary sensors are a breathing sensor (or breath rate sensor) that is configured to determine a breathing rate of the subject and/or whether the subject is breathing and a blood pressure sensor configured to determine a blood pressure of the subject. The breathing rate and the blood pressure are further examples of parameters that are indicative of whether the subject is conscious and/or responsive. To determine the state of consciousness, the controller may for example be configured to determine whether the breathing rate is below a threshold (e.g. a pre-defined threshold) and/or whether the blood pressure is below a threshold (e.g. a pre-defined threshold).

Yet other examples for auxiliary sensors are an acceleration sensor configured to determine an acceleration and/or a direction of gravity and a position sensor (e.g. a satellite-based position sensor such as a Global Positioning System (GPS) sensor) configured to determine a position, in particular a position of the position sensor and/or of the device. To determine the state of consciousness, the controller may for example be configured to determine whether the acceleration experienced by the acceleration sensor and/or the device exhibits one or more characteristic features that may be associated with a fall of the user/subject (e.g. the user collapsing to the ground), for example that the acceleration is above a threshold (e.g. a pre-defined threshold), exhibits a sudden peak and/or is below a threshold (which may e.g. indicate that the user is lying on the ground motionless). Similarly, if the position of the device does not change over an extended period of time, this may also indicate that the user has become unconscious or unresponsive.

Further examples for auxiliary sensors are a hydration sensor, a body temperature sensor, a lactate sensor, a blood alcohol sensor, a carbon monoxide sensor, an urea sensor, a creatinine sensor, an albumin sensor, a bilirubin sensor and a hemoglobin sensor. Some or all of the auxiliary sensors may for example be implemented as optical sensors working in the visible spectrum and/or in the infrared spectrum. Measurements of the auxiliary sensors may be combined with analyte measurements (e.g. glucose measurements) and/or may contribute to an accurate detection of the analyte (e.g. a determination of the glucose level).

The device may comprise some or all of the one or more auxiliary sensors. Additionally or alternatively, some or all of the one or more auxiliary sensors may be external sensors, i.e. sensors of an external device, for example of a portable computing device such as a smartphone. The controller may for example be configured to obtain sensor signals and/or parameter values from external sensors using the communication unit, e.g. via a wireless communication means.

Additionally or alternatively, the controller may be configured to determine a concentration of the analyte in the tissue based on the response signal generated by the detection device and to determine the state of consciousness based on the determined concentration. The controller may for example determine whether the determined concentration (and/or a rate of change of the concentration) is below a first threshold or above a second threshold. The subject may for example be assumed to be unconscious and/or unresponsive if the concentration is below the first threshold or above the second threshold for more than a pre-defined amount of time (e.g. for more than one minute) and/or for more than a pre-defined number of analyte measurements (e.g. for three or more successive analyte measurements).

Preferably, the controller is configured to determine the state of consciousness based on a combination of sensor signals from the one or more auxiliary sensors and the response signal from the analyte measurement, e.g. based on the determined concentration and on one or more parameters measured by the auxiliary sensors. The controller may for example be configured to determine the state of consciousness based on a combination of two or more, in some examples of three or more, in one example of four or more of the determined concentration, the pulse, the oxygen saturation, the breathing rate, the blood pressure, the acceleration and the position. The controller may for example be configured to determine the state of consciousness based on at least the determined concentration and the pulse, the determined concentration and the oxygen saturation, the determined concentration and the acceleration and/or any combination of these four parameters. In some examples, the controller may determine the state of consciousness based on sensor signals from the one or more auxiliary sensors, but not based on the response signal. Performing an analyte measurement may require a lot of energy, e.g. for the excitation radiation source, and the auxiliary sensors may thus be more suitable for continuously monitoring the state of consciousness.

In some embodiments, the controller may further be configured to take into account user inputs to the device for determining the state of consciousness. For example, the controller may assume that the subject is conscious and responsive when the device receives user inputs. In some examples, the controller may request a user input, e.g. as a confirmation that the user is conscious and responsive.

The communication unit may comprise a display for displaying the monitoring information, the first aid instruction and/or the medical information, for example a liquid-crystal display, in particular an organic light-emitting diode (OLED) display. Additionally or alternatively, the communication unit may comprise a loudspeaker for acoustic playback of the monitoring information, the first aid instruction and/or the medical information. The display and/or the loudspeaker may for example be arranged on or in a main housing of the device. In one example, the loudspeaker may be a headphone.

The communication unit may comprise a wireless communication module that is configured to make an emergency call, e.g. via a cellular network and/or a satellite communication channel. The controller may for example be configured to control the communication unit to make an emergency call in response to the state of consciousness indicating that the subject is unconscious and/or unresponsive. In the context of this disclosure, the term "emergency call" may for example refer to any type of call, message or signal to an emergency contact point such as an emergency call center, a public safety answering point and/or one or more pre-defined emergency contacts (e.g. family members and/or friends). Additionally or alternatively, the wireless communication module may be configured to communicate (e.g. transmit and/or exchange data) with an external device, in particular a portable computing device such as a smartphone, for example via a wireless network such as Wi-Fi and/or via a short-range wireless technology such as Bluetooth.

The controller may be configured to transmit the monitoring information, the first aid instruction and/or the medical information to the external device via the wireless communication module. Preferably, the controller is further configured to cause the external device to provide the monitoring information, the first aid instruction and/or the medical information by acoustic, optical and/or wireless communication means of the external device, e.g. by sending corresponding commands or instructions to the external device. In this way, one may take advantage of communication means provided by the external device without having to provide such communication means as part of the device according to the invention. The controller may for example be configured to display the monitoring information, the first aid instruction and/or the medical information on a display of the external device. Additionally or alternatively, the controller may be configured to playback the monitoring information, the first aid instruction and/or the medical information via a loudspeaker of the external device and/or to provide the monitoring information, the first aid instructions and/or the medical information by wireless communication means of the external device. In a preferred embodiment, the controller is configured to cause the external device to make an emergency call, e.g. in response to determining that the subject has become unconscious or unresponsive.

In a preferred embodiment, the controller is configured to change from a first means for providing the monitoring information (e.g. a first one of said acoustic, optical, and/or wireless communication means) to a second means for providing the monitoring information (e.g. a second one of said acoustic, optical, and/or wireless communication means) based on the state of consciousness, the response signal and/or the sensor signal from the one or more auxiliary sensors. The controller may for example be configured to provide the monitoring information by optical means, e.g. on a display, during normal operation, which may be less intrusive than for example acoustic means. If the state of consciousness indicates that the subject is unconscious and/or unresponsive, the controller may for example switch to acoustic means such as a loudspeaker for providing the monitoring information. In some examples, the controller may be configured to already switch to the second means if the controller determines based on the state of consciousness, the response signal and/or the sensor signal from the one or more auxiliary sensors that a medical anomaly or issue has occurred (e.g. that the subject is about to become unconscious and/or unresponsive). The controller may for example be configured to determine that a medical anomaly or issue has occurred based on corresponding thresholds for the determined concentration and/or the parameters determined by the auxiliary sensors and/or based on thresholds for a rate of change of the determined concentration and/or of the parameters determined by the auxiliary sensors.

In a preferred embodiment, the controller is configured to repeatedly perform analyte measurements by controlling the excitation radiation source to irradiate excitation radiation into the tissue to be absorbed by the analyte contained therein and to control the detection device to detect said physical response and to generate a response signal indicative of the degree of absorption of said excitation radiation. This may for example allow for continuously monitoring a concentration of the analyte. The controller may for example be configured to perform analyte measurements at pre-defined (e.g. user-defined) points in time and/or with a pre-defined (e.g. user-defined) frequency, for example once per minute or once per hour.

Additionally or alternatively, the controller may also be configured to perform analyte measurements in response to certain events, in particular in response to the state of consciousness indicating that the subject has become unconscious and/or unresponsive (which may e.g. have been determined using the auxiliary sensors) and/or in response to a medical anomaly or issue having occurred, for example in response to determining that the subject is about to become unconscious and/or unresponsive.

The controller may further be configured to adjust a delay time between subsequent analyte measurements dynamically, in particular based on the state of consciousness, the response signal and/or the sensor signal from the one or more auxiliary sensors. The controller may for example be configured to adjust the time delay (e.g. decrease the time delay and thus increase the frequency of analyte measurements) in response to the state of consciousness indicating that the subject has become unconscious and/or unresponsive (which may e.g. have been determined using the auxiliary sensors) and/or in response to a medical anomaly or issue having occurred. In one example, the controller is configured to perform analyte measurements with a first time delay (or first frequency, e.g. once per hour) in a normal state (e.g. when the state of consciousness indicates that the subject is conscious and responsive) and with a second time delay that is smaller than the first time delay (or with a second frequency that is larger than the first frequency, e.g. once per minute) in an emergency state (e.g. when the state of consciousness indicates that the subject is unconscious and/or unresponsive).

The controller may be configured to record a time trace of a concentration of the analyte in said tissue, e.g. based on a plurality of analyte measurements. In some embodiments, said analyte is glucose and the controller is configured to record a time trace of the concentration of glucose in the tissue. The controller may be configured to determine a point in time of a most recent food intake by the subject based on the time trace of the concentration of glucose in the tissue. The controller may for example be configured to identify one or more characteristic features in the time trace that are associated with a food intake, for example an increase in the glucose level (e.g. above a threshold), in particular a sudden increase followed by a plateau and/or a slow decrease. In some examples, said point in time of the most recent food intake may constitute the monitoring information or may be comprised in the monitoring information. The controller may also be configured to determine the first aid instructions based on said point in time, e.g. when determining whether or not to recommend giving food to the subject.

Disclosed is further a method for monitoring an analyte in tissue of a human or animal subject using a device according to any one of the embodiments described herein. The method comprises (1) irradiating excitation radiation at two or more wavelengths of said plurality of wavelengths into the tissue to be absorbed by the analyte contained therein using the excitation radiation source, detecting the physical response of the measurement body or a component included therein and generating a response signal indicative of the degree of absorption of said excitation radiation using the detection device; (2) determine a state of consciousness of the subject that characterizes whether the subject is conscious and/or responsive, wherein the state of consciousness is determined based on said response signal and/or based on a sensor signal from one or more auxiliary sensors configured to measure a parameter indicative of whether the subject is conscious and/or responsive; and (3) if the state of consciousness indicates that the subject is unconscious and/or unresponsive, provide monitoring information pertaining to the degree of absorption of said excitation radiation and/or a first aid instruction based on the degree of absorption of said excitation radiation via the communication unit by one or more of said acoustical, optical, and/or wireless communication means.

The above numbering is for clarity reasons only and does not imply a certain order of execution of the method. As far as technically feasible, the method may be executed in an arbitrary order and parts thereof may be executed simultaneously at least in part. For example, the analyte measurement (i.e. irradiating excitation radiation into the tissue, detecting the physical response of the measurement body and generating the response signal) may be performed prior to or after determining the state of consciousness and in particular in response to determining that the subjects is unconscious and/or unresponsive.

The method may be executed by the controller of the device at least in part, preferably in its entirety, wherein the controller may e.g. control the components of the device accordingly to perform the respective actions or steps.

The analyte measurement may be performed as detailed above. The excitation radiation may in particular be irradiated into the tissue at two or more analyte-characteristic wavelengths, in some examples at three or more analyte-characteristic wavelengths, in one example at five or more analyte-characteristic wavelengths. The analyte measurement may comprise determining the concentration of the analyte in the tissue based on the response signal generated by the detection device.

The state of consciousness may for example be determined as described above, e.g. based on a pulse and/or an oxygen saturation of the subject and/or based on an acceleration. In some examples, the response signal, in particular the concentration of the analyte may also be taken into account for determining the state of consciousness.

In response to determining that the subject is unconscious and/or unresponsive, emergency information is provided via the communication unit, for example by providing the monitoring information and/or one or more first aid instructions based on the degree of absorption of the excitation radiation (i.e. based on the result of the analyte measurement) as detailed above. This may in particular comprise playing back or announcing the monitoring information and/or the one or more first aid instructions on a loudspeaker of the device or of an external device coupled to the device via the communication unit and/or making an emergency call, wherein the monitoring information may be provided as part of said emergency call.

In some embodiments, the method comprises determining a concentration of the analyte in the tissue based on the response signal generated by the detection device and the monitoring information quantifies the determined concentration of the analyte and/or indicates whether the determined concentration is below or above one or more thresholds, e.g. as described above. Additionally or alternatively, the first aid instructions may be determined depending on whether the determined concentration is below or above one or more thresholds, e.g. by providing a first first aid instruction when the determined concentration of above a threshold and a second first aid instruction different from the first first aid instruction when the determined concentration is below the threshold.

The method may further comprise providing medical information pertaining to a general state of health of the subject, in particular to one or more medical conditions of the subject, via the communication unit if the state of consciousness indicates that the subject is unconscious and/or unresponsive, for example as described above.

In some embodiments, the monitoring information, the first aid instruction and/or the medical information may be provided through an external device, e.g. by making use of communication means provided by said external device, either in addition to or instead of communication means of the device itself. The method may comprise transmitting the monitoring information, the first aid instruction and/or the medical information to an external device, which may for example be a portable computing device such as a smartphone. The method may further comprise causing the external device to display the monitoring information, the first aid instruction and/or the medical information on a display of the external device. Additionally or alternatively, the method may also comprise causing the external device to playback the monitoring information, the first aid instruction and/or the medical information via a loudspeaker of the external device. Additionally or alternatively, the method may also comprise causing the external device to provide the monitoring information, the first aid instruction and/or the medical information by wireless communication means of the external device, e.g. to an emergency call center or an ambulance. In some examples, the method may also comprise causing the external device to make an emergency call, e.g. in response to the state of consciousness indicating that the subject has become unconscious and/or unresponsive.

In a preferred embodiment, the method further comprises providing the monitoring information via the communication unit by a first means for providing the monitoring information, in particular optical means (e.g. on a display of the device or of an external device), if the state of consciousness indicates that the subject is conscious and/or responsive. This may be a normal state or mode of operation of the device. If the state of consciousness indicates that the subject has become unconscious and/or unresponsive, the monitoring information may be provided via the communication unit by a second means for providing the monitoring information, in particular acoustic means, either in addition to or instead of the first means (i.e. the means of communication may be changed or switched). This may be an emergency state or mode of operation of the device. Preferably, the second means is not used for providing the monitoring information in the normal state.

In some embodiments, the method may comprise repeatedly performing analyte measurements, i.e. by irradiating excitation radiation into the tissue to be absorbed by the analyte contained therein using the excitation radiation source, detecting the physical response of the measurement body or a component included therein and generating a response signal indicative of the degree of absorption of said excitation radiation using the detection device. The analyte measurements may be performed automatically, i.e. without any user input or interference. The analyte measurements may be performed with a first time delay between subsequent measurements (e.g. corresponding to a first frequency of analyte measurements, which may e.g. be once per hour). The first time delay or first frequency may for example be used in the normal state. If the state of consciousness indicates that the subject has become unconscious and/or unresponsive (e.g. in the emergency state), the time delay between subsequent measurements may be adjusted to a second time delay, the second time delay being smaller than the first time delay (for example by increasing the frequency of analyte measurements to a second frequency that is larger than the first frequency, e.g. once per minute).

In some embodiments, no analyte measurements may be performed automatically in the normal state (but e.g. only in response to a corresponding user command or input). In response to the state of consciousness indicating that the subject has become unconscious and/or unresponsive, one or more analyte measurements may be performed automatically, e.g. with the second time delay or second frequency.

In some embodiments, the method may further comprise determining whether a medical anomaly or issue has occurred, e.g. as described above. The method may also comprise switching from the first means for providing the monitoring information to the second means in response to detecting a medical anomaly or issue. Additionally or alternatively, the method may also comprise performing an analyte measurement and/or changing a time delay between or a frequency of analyte measurements in response to detecting a medical anomaly or issue. Additionally or alternatively, some or all of the aforementioned actions may be taken based on corresponding thresholds for the determined concentration and/or for the parameters determined by the auxiliary sensors and/or based on thresholds for a rate of change of the determined concentration and/or of the parameters determined by the auxiliary sensors, for example if a rate of change of one or more of these quantities (e.g. of the pulse and/or of the determined concentration) exceeds a respective threshold.

In some embodiments, the method may comprise temporarily increasing a contact pressure between the measurement body and the skin of the subject, e.g. prior to performing an analyte measurement. The contact pressure may for example be increased by one or more of reducing an inner diameter of the device, reducing an inner circumference of the device and moving the measurement body radially inward towards the body part that the device is secured to, e.g. as described above. The analyte measurement may be performed while maintaining the temporarily increased contact pressure. Afterwards, the temporarily increased contact pressure may be reduced again (e.g. released completely) while the device remains secured to the body part of the subject.

The solution provided by the present invention is not limited to a particular response to the state of consciousness indicating that the subject is or has become unconscious and/or unresponsive. In some examples, other actions may be taken instead of or in addition to providing the monitoring information and/or the first aid instruction as described above. For example, an emergency call may be made and/or an acoustic alarm may be played, in some examples without providing monitoring information and/or first aid instructions.

SHORT DESCRIPTION OF THE FIGURES

In the following, a detailed description of the invention and exemplary embodiments thereof is given with reference to the figures. The figures show schematic illustrations of

Fig. 1: the measurement principle underlying embodiments of the invention;
Fig. 2: the absorption spectrum of glucose in water, with the water background subtracted;
Fig. 3: a sectional view of an apparatus suitable for carrying out embodiments of the invention relying on response signals based on a deflection of a detection light beam;
Fig. 4: results of a Clarke's error grid analysis obtained with an apparatus of the type shown in Fig. 3;
Fig. 5: an apparatus suitable for carrying out embodiments of the invention relying on response signals based on piezoelectric responses to heat or pressure waves received from the tissue subjected to the analysis;
Fig. 6: an apparatus suitable for carrying out embodiments of the invention relying on response signals based on interferometrically detected phase changes in a detection light beam;
Fig. 7: a device for monitoring an analyte in tissue of a human or animal subject according to an exemplary embodiment of the invention;
Fig. 8: a main housing of a device for monitoring an analyte in tissue of a human or animal subject in accordance with an exemplary embodiment of the invention; and
Fig. 9: a flow chart of a method for monitoring an analyte in tissue of a human or animal subject not according to an exemplary embodiment of the invention.

DESCRIPTION OF THE PREFERRED EMBODIMENTS

It is to be understood that both the foregoing general description and the following description are exemplary and explanatory only and are not restrictive of the methods and devices described herein. In this application, the use of the singular may include the plural unless specifically state otherwise. Also, the use of "or" means "and/or" where applicable or unless stated otherwise. Those of ordinary skill in the art will realize that the following description is illustrative only and is not intended to be in any way limiting. Other embodiments will readily suggest themselves to such skilled persons having the benefit of this disclosure. Reference will now be made in detail to various implementations of the example embodiments as illustrated in the accompanying drawings. The same reference signs will be used to the extent possible throughout the drawings and the following description to refer to the same or like items.

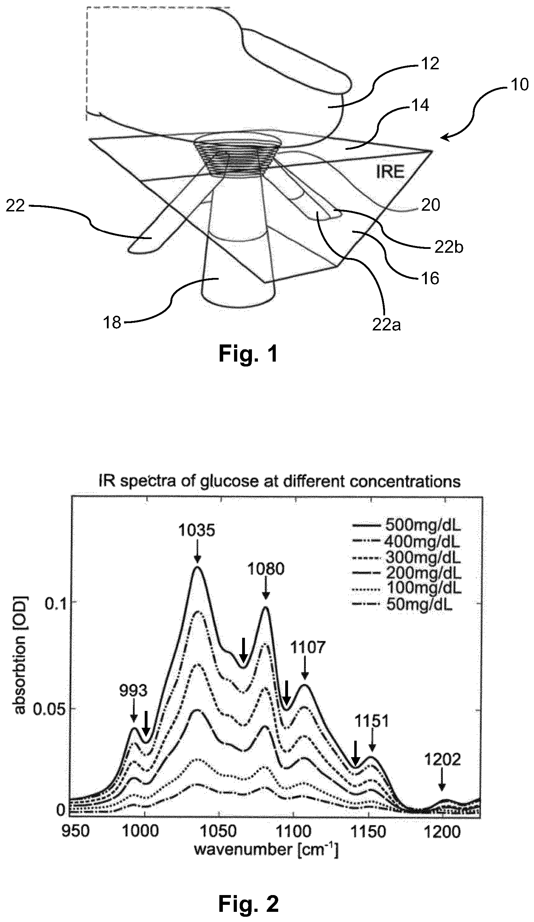

Fig. 1 is a schematic illustration of the measurement principle underlying the analyte measurement procedure summarized above and described in more detail in the following. While the device of the invention is suitable for analyzing various types of tissue of both human and animal subjects comprising at least one analyte, the following description will focus on specific embodiments, where the tissue is the skin of a patient and the analyte is glucose within the interstitial fluid of the skin. It is to be understood that all details and explanations given in the following with specific reference to glucose measurement are considered in relation to other types of tissue and analytes as well, where applicable, without explicit mention in the following.

In the illustration of Fig. 1, a fingertip 12 of a user is brought in thermal contact with a contact surface 14 of a measurement body 16. In an alternative implementation, which is not shown, the fingertip may be coupled acoustically to the measurement body through an acoustic cell which may include a hollow space filled with a liquid or gas allowing for a transfer of pressure waves to the measurement body.

An excitation beam 18 is irradiated to the measurement body 16 through air or through a waveguide (not shown) and then through the measurement body 16 and into the skin at the fingertip 12. In order to determine the concentration of the glucose in the skin, in particular in the interstitial fluid of the skin, various wavelengths of the excitation radiation 18 are chosen one after the other or at least partially at the same time for absorption measurement, such that from the measured absorption values the concentration of the glucose can be determined.

In Fig. 2, absorption spectra are shown for different concentrations of glucose in water, wherein the contribution of the absorption by water has been subtracted. As is seen therein, the glucose molecule has several characteristic absorption peaks in the mid infrared region at wave numbers ranging between 993 $cm^{-1}$ and 1202 $cm^{-1}$, corresponding to wavelengths ranging from 10.07 $\mu$m to 8.32 $\mu$m, respectively. In between adjacent absorption peaks, local absorption minima are seen, which are indicated by vertical arrows without wave numbers in Fig. 2. As is apparent from Fig. 2, particularly the difference in absorption at the absorption peaks and the local absorption minima are characteristic for the glucose concentration. Accordingly, in order to be able to determine the glucose concentration, it is preferable to measure the absorption at some or all of the absorption peaks and at some or all of the local absorption minima and potentially also at some point between the maxima and minima. These wavelengths are referred to as a "analyte(glucose)-characteristic wavelengths" herein. Note, however, that the absorptivity at the lowest local minimum at 1140 cm-1 is still 18% of the absorptivity at the highest peak at 1035 cm-1 in the relevant part of the spectrum. Accordingly, the absorption at any of these wavelengths will noticeably depend on the concentration of the glucose, such that these wavelengths are characteristic for the glucose, i.e. "glucose-characteristic wavelengths". In contrast to this, at about 1180 cm-1, the absorptivity is practically zero, and hence a global rather than a local minimum, and this wavelength is obviously not characteristic for the glucose (but may e.g. be used for reference measurements, for example to determine a contact pressure between the measurement body 16 and the skin of the fingertip 12). While wavelengths exactly at the absorption peaks or local absorption minima are the preferred choices for the glucose-characteristic wavelengths, wavelengths close to the peaks/local minima but in an individually defined distance from them may also be used. Accordingly, as understood herein, "analyte-characteristic wavelengths" are also wavelengths where the difference in absorption to that at the closest absorption peak or the closest local absorption minimum is less than 30%, preferably less than 20% of the difference in absorption between the closest absorption peak and closest local absorption minimum.

The intensity of the excitation beam 18 is time modulated with a certain frequency f, such that the excitation radiation, in this case excitation light, has alternating intervals of high intensity and low or even vanishing intensity. Without wishing to limit the modulation to any particular waveform, high intensity intervals are referred to as "excitation light pulses" in the following. During the excitation light pulses, excitation light having the glucose-characteristic wavelength will be absorbed, such that the radiation energy will be converted to heat. Since the glucose molecules relax from the excited state within approximately $10^{-12}$ s, the generation of a corresponding heat pulse and/or pressure wave can be regarded as occurring instantaneously for all practical purposes.

Accordingly, along with the excitation light pulses, local heat pulses are generated at the absorption site, leading to a temperature field that varies as a function of space and time and that could be referred to as a thermal wave. As was explained above, the term thermal "wave" is somewhat misleading, since the travel of heat through the material is not governed by a wave equation, but by a diffusion equation instead. However, the notion of a "heat wave" is correct at least to the extent that heat pulses propagate from within the skin to the surface 14 of the measurement body 16 and into the measurement body 16 similarly to what one is used to from wave propagation. A thermal gradient 20 that is caused by such a heat pulse is schematically shown in Fig. 1.

The heat received by the measurement body 16 from the skin of the fingertip 12 causes a physical response that can be detected with one of various possible detection devices which are devised for generating a response signal based on the physical response, wherein this response signal is indicative of the degree of absorption of the excitation light. Various ways of detecting the physical response and generating suitable response signals will be described below.

However, irrespective of the precise way of detecting the physical response, it is worth noting that the maximum depth underneath the surface of the skin in which the absorption can be detected by means of heat pulses travelling to the measurement body 16 is found to be limited to a good approximation by the thermal diffusion length $\mu_t$ of the skin, which is defined as

$$\mu t(f) = \sqrt{\frac{k_t}{\rho \cdot C_p \cdot 2\,f}}$$

and which depends on the density $\rho$, the specific heat capacity $C_p$, and the thermal conductivity $k_t$ of the tissue as well as on the modulation frequency f of the excitation light. In other words, by selecting the modulation frequency f, a depth can be defined up to which any absorption of the excitation light is reflected in the heat pulses received at the measurement body 16.

With reference again to Fig. 1, in the embodiment shown, the physical response to the absorption heat received from the skin is a change in refractive index in an area close to the surface 14 of the measurement body 16 where the heat gradient 20 is transiently formed. This local change in refractive index forms what could be regarded as a thermal lens that can be detected by means of a detection light beam 22. The detection beam 22 is passing through the thermal lens or heat gradient region and then reflected at the interface of the measurement body 16 and the skin of the Fig. 12. Whenever a heat pulse is received from the skin, a local change in refractive index occurs, and this leads to a deflection of the detection beam 22 by the interaction with the material of the measurement body in the region of the thermal lens. In Fig. 1, reference sign 22b corresponds to the non-deflected detection beam 22, whereas reference sign 22a corresponds to the detection beam when it is deflected due to the thermal lens formed in the heat gradient region 20. This deflection can be measured and forms an example of the aforementioned response signal. The degree of the deflection is indicative of the amount of heat received, and hence the degree of absorption of the excitation light 18 in the skin of the finger 12.

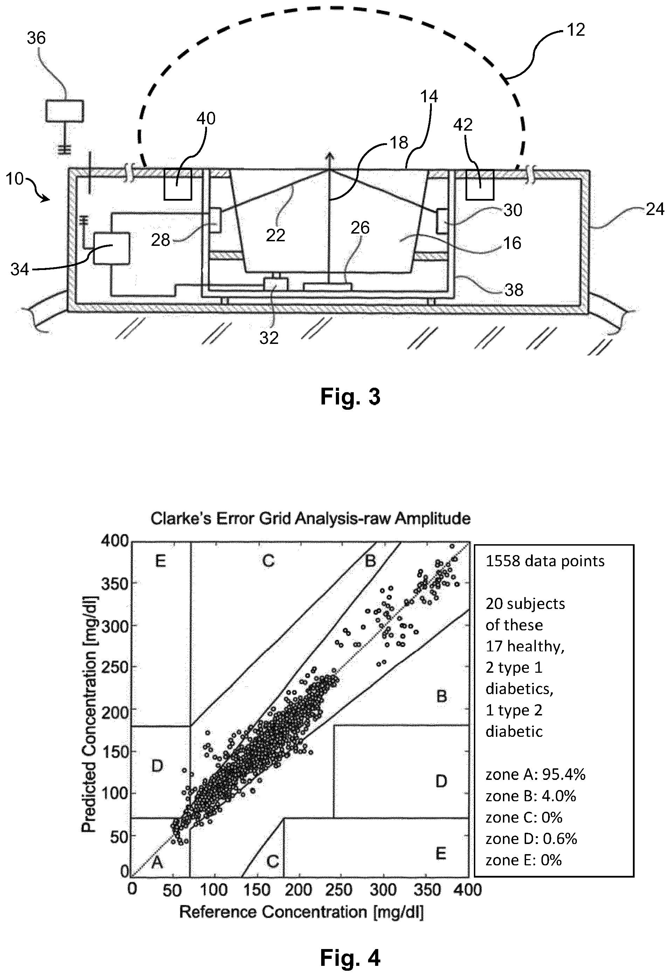

Fig. 3 shows a more detailed sectional view of an apparatus 10 that relies on the measurement principle as illustrated with reference to Fig. 1. The apparatus 10 comprises a housing 24 which includes the measurement body 16, having a top surface (contact surface) 14 on which a body part of the subject/patient such as a finger 12 rests. Within the housing 24, an excitation light source (excitation radiation source) 26 is provided, which generates the excitation light beam 18. In the embodiment shown, the excitation light source 26 comprises an array of quantum cascade lasers (not shown) each having a dedicated wavelength. For example, the array of quantum cascade lasers could include individual quantum cascade laser elements with wavelengths corresponding to the absorption peaks and local minima shown in Fig. 2 (i.e. the glucose-characteristic wavelengths), as well as other wavelengths that can be used for reference measurements (e.g. for determining a contact pressure between the measurement body 16 and the skin of the fingertip 12, for example one or more characteristic wavelengths of water), or for detecting other substances that could be disturbing to the measurement of the glucose, for example lactate or albumin.

The apparatus 10 further comprises a light source 28, for example a laser, for emitting the detection beam 22, as well as a position-sensitive detector or sensor 30 (e.g. a quadrant photodiode) which allows for detecting the deflection of the detection beam 22. The light source 28 and the position-sensitive detector 30 together form an example of a detection device for detecting the physical response of the measurement body 16 to heat and/or pressure waves received from the fingertip 12. The measurement body 16 in this case is transparent for both, the excitation light beam 18 as well as the detection light beam 22.

In addition, a camera 32 or another imaging device is provided that allows for taking images of the contact surface 14 of the optical medium 16, to thereby record a skin pattern such as a fingerprint of the finger 12 resting on the contact surface 14. This skin pattern can be processed by a control unit 34 such as to identify and/or authenticate a user via his or her skin pattern (e.g. fingerprint).

The control unit 34 also serves for controlling the light sources 26 and 28 for the excitation light and the detection light, respectively, as well as the sensor 30. The control unit 34 forms an example of a "controller" as referred to above. The control unit 34 may also be in wireless connection with an external data processing device 36 to exchange data. For example, via the wireless connection, user-specific calibration data can be retrieved by the control unit 34 for the user that is identified via the fingerprint. In some examples, the control unit 34 and the external data processing device 36 may together form an example of a "controller" as referred to above. The controller can be comprised by one or more processors, microcontrollers, computers, ASICs, FPGAs, or the like. The controller may be distributed, as indicated in Fig. 3, with various components in data communication with each other, or could be formed by a single control unit, such as the control unit 34, which would be devised for all of the control functionalities described herein. The controller may be generally embodied in hardware, in software, or a combination of both.

As is further seen in Fig. 3, the excitation and detection light sources 26 and 28, as well as the position sensitive detector 30 are all attached to a common carrier structure 38. This means that these components can be preassembled with precision on this structure 38, such that they do not need to be individually adjusted or calibrated when assembling the apparatus 10.

In addition, the apparatus 10 comprises a corneometric device 40 that allows for measuring the water content of the skin. Corneometric devices for measuring the water content in the upper layer of the skin are per se known in the art and need not be described in detail here. For example, known corneometric devices measure the impedance, in particular capacitive impedance of the skin using two interdigital electrodes to which an AC voltage is applied. The corneometric device 40 of Fig. 3 is in contact with the fingertip 12 when the latter rests on the contact surface 14 of the measurement body 16. The corneometric device 40 is an example of the aforementioned "auxiliary sensors", i.e. a sensor that is per se unrelated to the measurement apparatus for measuring the analyte absorption.

The apparatus also comprises a pH-sensor 42 for measuring the pH value of the skin. pH sensors for measuring the pH value on surfaces, including those of the skin are per se known from prior art and need not be described in detail herein. pH sensors for measuring the pH value of the skin are commercially available for medical but also for cosmetic purposes.

Fig. 4 shows results of a Clarke's error grid analysis obtained with an apparatus of the type shown in Fig. 3, illustrating that with the measurement procedure described with reference to Fig. 1 to 3, indeed very reliable blood sugar concentrations can be measured in a purely non-invasive manner. The data shown in Fig. 4 are taken from Wo 2017/09782 A1.

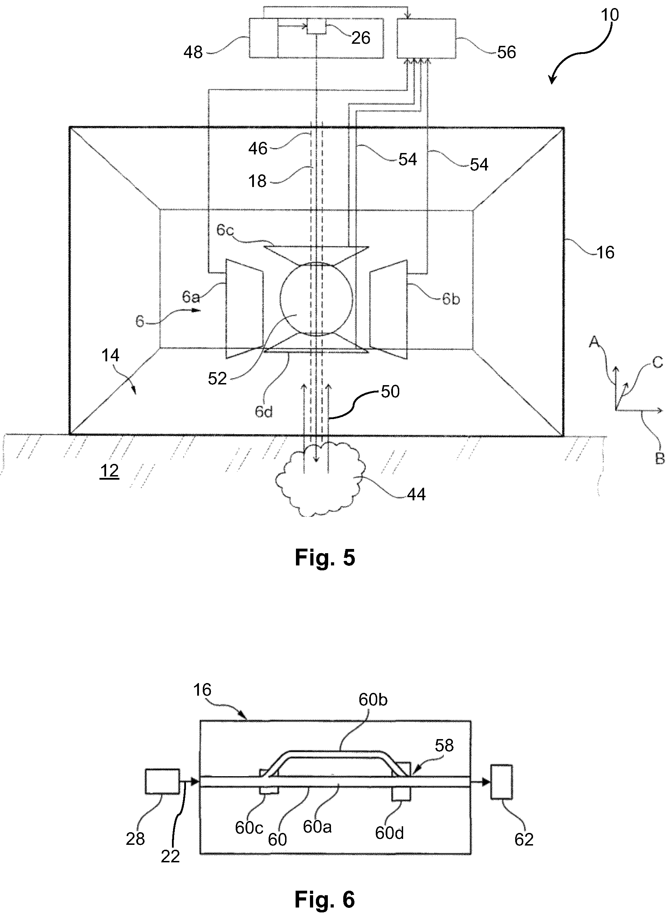

Fig. 5 schematically shows an apparatus 10 which relies on the same general principle involving absorption of excitation radiation for generating heat pulses received by the measurement body 16 from the tissue of the fingertip/body part 12 as that of Fig. 1 and 3, but differs in the physical response exploited and in the way the corresponding response signals are generated. Such an apparatus 10, as well as a large number of variations thereof are described in detail in Wo 2019/11059782, such that a detailed description may be omitted herein. As before, the apparatus comprises a measurement body 16 having a surface 14 which is brought in contact or coupling with the skin of a finger 12. Also, a source 26 for an excitation light beam 18 with modulated intensity is provided, which is irradiated into a region 44 underneath the surface of the skin 12 and absorbed therein. In this embodiment, the excitation light beam 18 runs through a bore 46 indicated by hashed lines through the measurement body 16, such that the measurement body 16 itself need not be transparent for it.

A control unit 48 is provided for modulating the intensity of the excitation light beam 18. This can generally be done in various ways, including a mechanical chopper or an element having a transmissivity or reflectivity that can be electronically controlled. However, in preferred embodiments, the intensity is modulated by modulating the on/off times of the excitation light source 26 as well as the operating current during the on-times thereof. The control unit 48 may for example be integrated into a controller such as the control unit 34 of Fig. 1 at least in part.

A thermal wave caused by the time varying absorption of the intensity modulated excitation beam 18 in the region 44 of the skin 12, which is symbolically represented by arrows 50, enters the measuring body 16 where it can be detected in a detection region 52 which has piezoelectric properties. Pressure changes associated with received heat 50 or pressure waves lead to electrical signals that can be recorded with electrodes 6a to 6d, which are connected via conducting leads 54 with an evaluation device 56 for analyzing the tissue (the skin of figer 12). The electrodes 6a to 6d, in some examples together with the evaluation device 56, may form an example of a detection device as referred to above. The evaluation device 56 may be a digital processing device, for example a microcontroller or processor or a computer. In some exmaples, the evaluation device may be integrated into a controller such as the control unit 34 of Fig. 1 at least in part. In this example, the change in pressure resembles the physical response of the measurement body 16, or other component included therein, to heat received from the tissue of the fingertip 12 upon absorption of the excitation radiation, which is detected using the piezoelectric properties of the measurement body 16 and the electrodes 6a to 6d, and which leads to electrical signals representing the response signal that is indicative of the degree of absorption of excitation radiation 18.

In alternative variants suggested by the present applicant, as for example disclosed in international application WO 2020/094265 A1, the detection device may comprise an interferometric device allowing for assessing said change in phase of a first part of the detection beam with respect to a second part of the detection beam, wherein only one of the parts of the detection beam passing through a measurement arm is affected by the effects of the heat or pressure wave in the measurement body 16, and generates a response signal indicative of said change in phase. In this example, the physical response of the measurement body 16 (or a component included therein) to heat received from said material 12 upon absorption of said excitation radiation 18 is again a local change in index of refraction, while the response signal is in this case an interferometric signal reflecting a change in the phase of the detection beam due to the local change in refractive index.

This is schematically illustrated in Fig. 6, where a measurement body 16 is shown which is to be brought in contact with the skin of a body part (such as the finger, not shown in Fig. 6). In this example, the measurement body 16 may be a silicon substrate in which a light guiding structure 58 is provided, which forms an interferometric device 60. The interferometric device 60 forms a Mach-Zehnder interferometer, having a measurement arm 60a and a reference arm 60b. Detection light 22 generated by a detection light source 28 is fed into the light guiding structure 58 and is splitted by a splitter 60c into a portion or a part of the detection beam travelling along the measurement arm 60a and a portion or part travelling along the reference arm 60b, which portions are then united by a combiner 60d. The measurement body 16 is used or arranged such that the reference arm 60a is exposed to heat received from the skin upon absorption of excitation light, but not, or at least to a much lesser extent, the reference arm 60b. Due to received heat, the refractive index in the measurement arm 60a will change, which in turn leads to a phase shift of the detection light 22 travelling along the measurement arm 60a. Since the light travelling along the reference arm 60b is unaffected by the heat received, there will be a change in relative phase of the two portions of light combined by the combiner 60d, which leads to an interference pattern that can be detected using a detector 62. Accordingly, the light source 28, the interferometric device 60 and the detector 62 may together form an example of a detection device as referred to above.

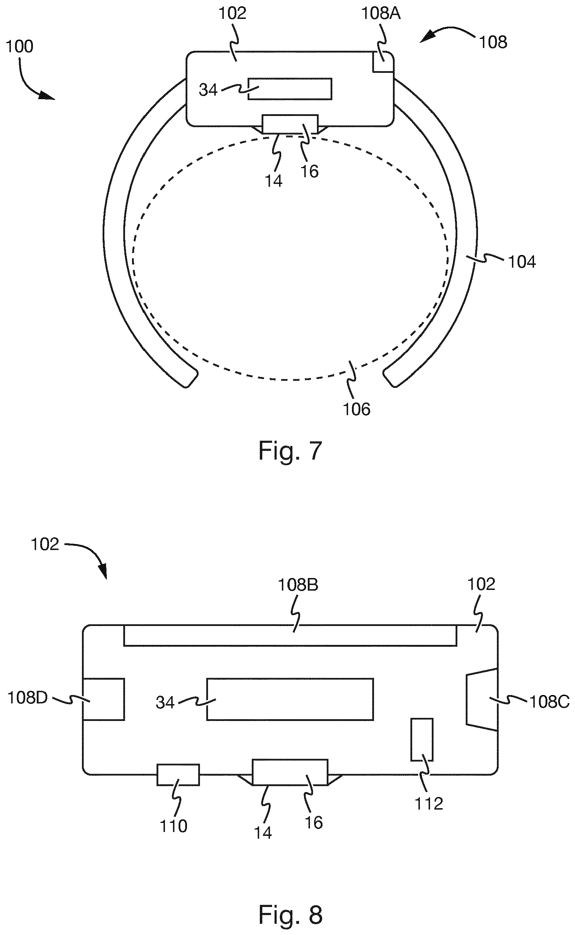

Fig. 7 shows a schematic illustration of a device 100 for monitoring an analyte in tissue of a human or animal subject according to an exemplary embodiment of the invention. The device 100 comprises a main housing 102 and a holding portion 104 for the securing the device 100 to a body part 106 of a human or animal subject, for example to an upper arm or a wrist of a user/patient. The holding portion 104 is configured to be arranged around the body part 106 so as to extend circumferentially around the body part 106 at least in part as illustrated in Fig. 7 or completely. The holding portion 104 may for example be a wristband or a similar structure. The device 100 further may further comprise a display (not shown), which may be arranged on or in an upper surface or wall of the main housing, e.g. similar to the display 108B in Fig. 8. In some examples, the device 100 may be a wearable device, in particular a smartwatch.

In the following, the direction extending azimuthally around the body part 106 is referred to as the circumferential direction and a direction that is perpendicular to the surface (i.e. the skin) of the body part 106 is referred to as a radial direction.

The device further comprises an apparatus for detecting the analyte (e.g. glucose) in tissue of the body part 106, e.g. in the skin, using a detection method as set forth above. The apparatus for detecting the analyte is arranged in the main housing 102 and may for example be similar to the apparatus of any one of Figs. 3, 5 and 6. In particular, the apparatus comprises a measurement body 16 having a contact surface 14 that is suitable to be brought in contact with the skin of the subject wearing the device 100 to permit heat and/or pressure waves generated by absorption of excitation radiation in said tissue of the body part 106 to be transferred to the measurement body 16. The apparatus further comprises an excitation radiation source (not shown) for irradiating excitation radiation at a plurality of analyte-characteristic wavelengths into the tissue and a detection device (not shown) for detecting a physical response of the measurement body 16 to heat and/or pressure waves received from the tissue upon absorption of the excitation radiation and for generating a response signal based on the detected physical response. The device 100 also comprises a controller 34 for controlling the excitation radiation source and the detection device to perform analyte measurements. The measurement body, the excitation radiation source, the detection device and the controller 34 may for example be embodied as described above with reference to Figs. 3, 5 and 6. In one example, the main housing 102 is similar to or corresponds to the housing 24 of the apparatus 10 of Fig. 3. In some examples, the controller 34 may be integrated into a main control unit of the device 100 at least in part, wherein the main control unit may for example control the display of the device 100 and may be configured to provide smartwatch capabilities as known in the art.

In the example of Fig. 7, the measurement body 16 is arranged in a bottom surface or bottom wall of the main housing 102 (e.g. opposite to the display of the device 100) such that the measurement body 106 protrudes from the bottom surface or wall. When the device 100 is secured to the body part 106, the contact surface 14 faces the body part 106. An interface between the bottom surface or wall and the measurement body 106 may be covered or sealed off by a sealing, e.g. a rubber sealing, which may for example cover or seal off exposed portions of the sidewalls of the measurement body 106. In some examples, the device 100 may further comprise actuated means (not shown) for temporarily increasing a contact pressure between the measurement body 16 and the skin of the subject (i.e. the skin of the body part 106). The actuated means may for example comprise an actuator (not shown), for example an electric actuator or a piezoelement, that is configured to move the measurement body 16 back and forth in the radial direction, i.e. radially inward towards the body part 106 and radially outward away from the body part 106 (i.e. down and up, respectively, in the example of Fig. 7). Additionally or alternatively, the actuated means may for example comprise one or more deformable members (not shown), for example one or more inflatable bodies, which may for example be configured to be deformed so as to reduce an inner diameter and/or an inner circumference of the holding portion 104 and/or of the device 100, e.g. by inflating the holding portion 104 or a part thereof.

The device 100 further comprises a communication unit 108 that is configured to provide monitoring information pertaining to the degree of absorption of the excitation radiation and/or a first aid instruction based on the degree of absorption of the excitation radiation by acoustic, optical, and/or wireless communication means. In the example of Fig. 7, the communication unit 108 comprises an optical indicator 108A, for example a light-emitting diode (LED). The optical indicator 108A may for example be configured to provide monitoring information pertaining to the degree of absorption of the excitation radiation by indicating whether a glucose level or concentration of the subject/user that was determined by one or more analyte/glucose measurements is below a predefined threshold, e.g. below 70 mg/dL, in some examples below 60 mg/dL, in one example below 50 mg/dL (i.e. to indicate whether the user is hypoglycemic or not). The device 100 may for example comprise a corresponding label (not shown) arranged adjacent to the optical indicator 108A that indicates or explains the meaning or function of the optical indicator 108A (e.g. a text label "hypoglycemia" or "hypoglycemic"). The optical indicator 108A may for example indicate that the glucose level is below the pre-defined threshold by lighting up permanently or by flashing repeatedly. In some examples, the optical indicator 108A may also be configured to indicate the glucose level itself, e.g. by adjusting a color of light emitted by the optical indicator 108A accordingly (e.g. green light if the glucose level is above a first threshold, e.g. above 70 mg/dL, yellow if the glucose level is below the first threshold, but above a second threshold, e.g. between 50 mg/dL and 70 mg/dL, and red if the glucose level is below the second threshold, e.g. below 50 mg/dL).

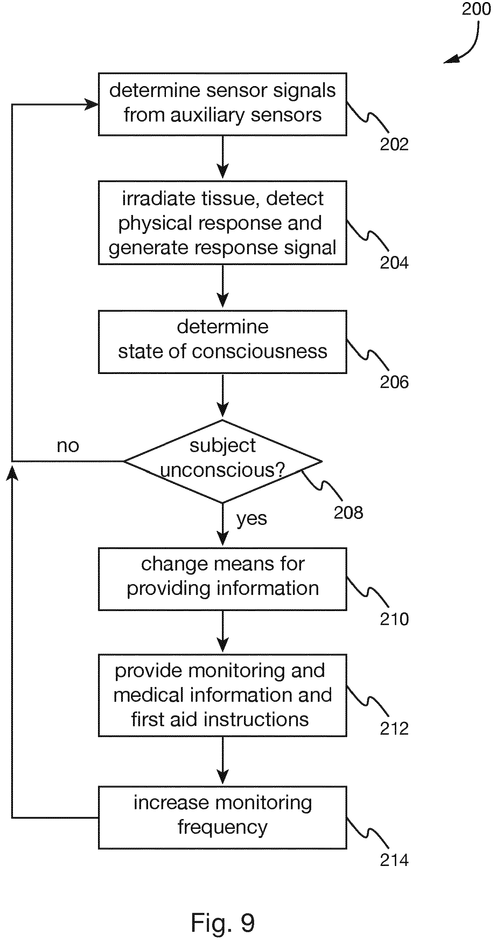

The controller 34 is configured to determine a state of consciousness of the subject that characterizes whether the subject is conscious and/or responsive. The controller may be configured to determine the state of consciousness based on the response signal (for example based on the glucose level) and/or based on a sensor signal from one or more auxiliary sensors (not shown) configured to measure a parameter indicative of whether the subject is conscious and/or responsive (for example a pulse). The controller 34 may in particular determine the state of consciousness as detailed below for the method 200 of Fig. 9, e.g. by executing steps 202 to 206.

The controller 34 is further configured to provide monitoring information pertaining to the degree of absorption of said excitation radiation and/or a first aid instruction based on the degree of absorption of said excitation radiation via the communication unit 108 (e.g. by controlling the communication unit 108 accordingly) by one or more of said acoustic, optical, and/or wireless communication means in response to the state of consciousness indicating that the subject is unconscious and/or unresponsive. In the example of Fig. 7, the controller 34 is configured to activate the optical indicator 108A (and optionally to adjust a color of the light emitted by the optical indicator 108A) if the controller 34 determines that the state of consciousness indicates that the subject is unconscious and/or unresponsive. The controller 34 may in particular be configured to determine the state of consciousness of the subject and to provide the monitoring information and/or the first aid instruction as detailed below for the method 200 of Fig. 9, e.g. by executing steps 208 to 214. In some embodiments, the controller 34 is configured to execute the method 200 at least in part, preferably in its entirety (i.e. all of the steps 202 to 214).

Fig. 8 depicts a schematic illustration of a main housing 102 of a device for monitoring an analyte in tissue of a human or animal subject in accordance with an exemplary embodiment of the invention. The main housing 102 may be used in a device according to any one of the embodiments described herein, for example as the main housing of the device 100 of Fig. 7. The main housing 102 houses an apparatus for detecting an analyte (e.g. glucose) as set forth above including a measurement body 16, an excitation radiation source (not shown), a detection device (not shown) and a controller 34, e.g. similar to the housing 24 of the apparatus 10 of Fig. 3. In other embodiments, some or all of the components of the main housing 102 may be arranged in other parts of the device that the main housing is used with, for example in the holding portion 104.

The main housing 102 further comprises a communication unit 108 for providing monitoring information pertaining to the degree of absorption of said excitation radiation and/or a first aid instruction based on the degree of absorption of said excitation radiation by acoustic, optical, and/or wireless communication means.

In the example of Fig. 8, the communication unit 108 comprises a display 108B (as an example for optical means), which is arranged on or in an upper surface or upper wall of the main housing 102, e.g. such that the display 108B faces away from the body part 106 when the device comprising the main housing 102 is secured to the body part 106. The display 108B may for example be a liquid-crystal display and in particular an organic light-emitting diode (OLED) display. The display 108B may be configured to display the result of one or more analyte/-glucose measurements, for example the most recent glucose level and/or a time trace of the glucose level. The display 108B may further be configured to display first aid instructions and/or medical information, for example as detailed below for method 200 of Fig. 9.

The communication unit 108 further comprises a loudspeaker 108C (as an example for acoustic means). The loudspeaker 108C is configured to playback monitoring information, first aid instructions and/or the medical information, e.g. to read the respective content aloud. The loudspeaker 108C may also be configured to play an acoustic alarm. The controller 34 may for example store one or more pre-recorded messages for playback on the loudspeaker 108C and/or may comprise a text-to-speech module (not shown) that is configured to convert text (e.g. values such as the analyte concentration and/or pre-defined messages) to speech.

The communication unit 108 also comprises a wireless communication module 108D, which may for example be or comprise a cellular network module configured to exchange data via a cellular network (e.g. a 4G and/or 5G cellular network) and/or a wireless network module configured to exchange data via a wireless network such as a Wi-Fi network. The wireless communication module 108D may be configured to make an emergency call, i.e. to call or message an emergency contact point such as an emergency call center and/or one or more pre-defined emergency contacts (e.g. family members or friends). The wireless communication module 108D may also be configured to exchange data with an external device such as a smartphone of the user. The wireless communication module 108D may in particular be configured to transmit the monitoring information, first aid instructions and/or the medical information to the external device and to cause the external device to provide some or all of this content, e.g. by using acoustic, optical and/or wireless communication means of the external device.

The main housing 102 further comprises a plurality of auxiliary sensors that are configured to measure parameters indicative of whether the subject is conscious and/or responsive. In the example of Fig. 8, the main housing 102 comprises a pulse and/or oxygen saturation sensor 110 that is configured to determine a pulse of the subject and/or an oxygen saturation level of blood of the subject. The pulse and/or oxygen saturation sensor 110 may for example be an optical pulse and/or oxygen saturation sensor as known in the art. The pulse and/or oxygen saturation sensor 210 may e.g. comprise one or more light sources (not shown) for illuminating the skin of the subject and one or more photodetectors (not shown) for detecting light scattered or reflected from the body part, which may be used to determine the pulse and/or the oxygen saturation. The pulse and/or oxygen saturation sensor 110 may be arranged on or in a bottom surface or wall of the main housing 102, e.g. adjacent to the measurement body 16, so as to face the body part 106 when device that the main housing 102 is used in is secured thereto. The pulse and/or the oxygen saturation may allow for determining whether the subject might be unconscious and/or unresponsive, e.g. if one or both of the pulse and the oxygen saturation fall below a respective threshold.

The main housing 102 further comprises an acceleration sensor or accelerometer 112 that is configured to determine an acceleration and/or a direction of gravity. The acceleration sensor 112 may for example be a piezo-based acceleration sensor and/or a microelectromechanical system-based (MEMS-based) acceleration sensor as known in the art. The acceleration sensor 112 may for example be configured to determine an acceleration along two or preferably three orthogonal axes. The acceleration sensor 112 may be configured to separate static (e.g. non-varying or slowly varying) and dynamic contributions to the acceleration, e.g. to distinguish a gravity-induced acceleration from a dynamic acceleration induced by movements of the user. The acceleration and/or the direction of gravity may also allow for determining whether the subject might be unconscious and/or unresponsive, for example if the user stops moving, if a sudden acceleration occurs and/or if a direction of gravity changes, e.g. because the user falls to the ground.

Additionally or alternatively, the main housing 102 and/or the device 100 may also comprise other auxiliary sensors (not shown), for example a breathing sensor configured to determine a breathing rate of the subject and/or whether the subject is breathing and/or a blood pressure sensor configured to determine a blood pressure of the subject. The breathing rate and the blood pressure are also examples of parameters that are indicative of whether the subject is conscious and/or responsive.

Fig. 9 shows a flow chart of a method 200 for monitoring an analyte in tissue of a human or animal subject not according to an exemplary embodiment of the invention. The method 200 may be executed with a device for monitoring an analyte in tissue of a human or animal subject according to any one of the embodiments described herein. In the following, the method 200 is described using the device 100 of Fig. 7 and the main housing 102 of Fig. 8 as a non-limiting example for illustration purposes. This is, however, not intended to be limiting in any way and the method 200 may also be executed using a different device. Furthermore, the method 200 is not limited to the order of execution implied by the flow chart in Fig. 9. As far as technically feasible, the method 200 may be executed in an arbitrary order and parts thereof may be executed simultaneously at least in part, e.g. steps 202 and 204 and/or steps 210 to 214.

In some examples, the method 200 may be executed by the controller 34 of the device 100 at least in part. Preferably, the controller 34 is configured to execute all of the steps of the method 200 and in particular automatically, i.e. without any user input or interference. The controller 34 may for example be configured to control the excitation radiation source, the detection device and the communication unit 108 to perform the respective actions.

The device 100 may be secured to a body part 106 of the subject such as to a wrist or an upper arm using the holding portion 104 either prior to execution of the method 200 or as part of the method 200. Preferably, the device 100 is secured such that the contact surface 14 of the measurement body 16 is in contact with the skin of the subject. In some examples, the method 200 may also comprise establishing contact and/or temporarily increasing a contact pressure between the measurement body 16 and the skin of the subject, for example by actively moving the measurement body 16 towards the body part 106, prior to performing an analyte measurement in step 204.

The method 200 comprises, in step 202, determining (e.g. generating and measuring) sensor signals from one or more auxiliary sensors that are configured to measure a parameter indicative of whether the subject is conscious and/or responsive. This may in particular comprise determining the respective parameters based on or from the corresponding sensor signals. For example, a pulse of the subject and/or an oxygen saturation of blood of the subject may be measured using the blood and/or oxygen saturation sensor 110. Additionally or alternatively, an acceleration and/or a direction of gravity may be determined using the acceleration sensor 112. Preferably, some or all of said parameters are measured continuously, e.g. with a pre-defined measurement frequency such as for example with a frequency between once every 1 ms and once every 10 s, e.g. once every 100 ms or once every 1 s. In some embodiments, the method 200 may not comprise generating and/or measuring the sensor signals of some or all of the auxiliary sensors, but the sensor signals (and/or the associated parameters) may e.g. already have been generated and measured prior to execution of the method 200 and may only be obtained in step 202, e.g. from a storage medium of the controller 34.

The method 200 further comprises, in step 204, performing an analyte measurement (e.g. a glucose measurement) as set forth above by irradiating excitation radiation at two or more analyte-characteristic wavelengths into the tissue of the body part 106, e.g. into the skin of the body part 106, to be absorbed by the analyte contained therein using the excitation radiation source 26. Using the detection device 28, 30, the physical response of the measurement body 16 or a component included therein is detected and a response signal indicative of the degree of absorption of said excitation radiation using the detection device is generated. Preferably, step 204 also comprises determining a concentration of the analyte in the tissue, e.g. a glucose level, based on the response signal. In some examples, the analyte measurement may be performed repeatedly with a first frequency, wherein the first frequency may e.g. be between once every 5 minutes and once every three hours, for example once every 15 minutes or once every hour.

In step 206, a state of consciousness of the subject is determined, wherein the state of consciousness characterizes whether the subject is conscious and/or responsive. The state of consciousness may be determined based on one or both of the response signal (e.g. based on the glucose level) and the sensor signals from the one or more auxiliary sensor 110, 112 (e.g. based on the pulse, the oxygen saturation and/or the acceleration). In some examples, the state of consciousness is determined based on the sensor signals from the one or more auxiliary sensors 110, but not based the response signal. Performing an analyte measurement may require more energy than performing measurements with the auxiliary sensors and may thus be performed less frequently. The one or more auxiliary sensors 110, 112 may therefore be better suited to continuously monitor the state of consciousness. In some examples, no analyte measurements are performed prior to determining that the subject is unconscious and/or unresponsive in step 208.

The state of consciousness may for example be determined by comparing the parameters measured by the auxiliary sensors to one or more thresholds. For example, the subject may be assumed to be unconscious if the pulse is below a pre-defined threshold for more than a pre-defined amount of time (e.g. below 30/min for more than 30 seconds). Additionally or alternatively, the state of consciousness may be determined based on a time trace of the pulse, e.g. by identifying one or more characteristic features of the subject becoming unconscious such as a sudden drop and/or a sudden spike in the pulse rate. A similar determination may also be made based on the oxygen saturation. In addition to or instead of these physiological parameters, non-physiological parameters such as the acceleration may be taken into account. The subject may for example be assumed to be unconscious if the acceleration exceeds a pre-defined threshold (e.g. as a result of the subject collapsing) prior to an extended period of no or negligible acceleration (e.g. as a result of the subject lying on the floor unconsciously). Preferably, a plurality of parameters are used in combination to determine the state of consciousness (e.g. pulse, oxygen saturation and acceleration), which may allow for determining the state of consciousness more reliably. In some examples, determining the state of consciousness may also comprise requesting a user input, for example by asking the user to confirm that the user is conscious and responsive (e.g. by touching a button or the display 108B).

In step 208, the state of consciousness is assessed to determine whether the subject is or has become unconscious and/or unresponsive. If the state of consciousness indicates that the subject is conscious and responsive, the method 200 may return to step 202 for continued monitoring of the subject. Otherwise, i.e. if the state of consciousness indicates that the subject is unconscious and/or unresponsive, the method 200 may proceed to step 210.

In response to the state of consciousness indicating that the subject is unconscious and/or unresponsive, a means for providing monitoring information pertaining to the degree of absorption of the excitation radiation, e.g. for providing the analyte/glucose concentration, may be changed in step 210. For example, as long as the subject is conscious and responsive, the analyte concentration may only be provided by optical means (first means), e.g. by displaying the analyte concentration on the display 108B. This may allow for providing the analyte concentration to the subject/user in an unintrusive way in non-emergency situations. If the subject becomes unconscious and/or unresponsive, a second means for providing the monitoring information, in particular an acoustic means, may be used in addition to or instead of the display 108B. For example, the analyte concentration may additionally be announced using the loudspeaker 108C, which may be more likely to be noticed by first responders in case of emergency.

In step 212, monitoring information pertaining to the degree of absorption of said excitation radiation and/or a first aid instruction based on the degree of absorption of said excitation radiation is provided via the communication unit 108 by one or more of said acoustic, optical, and/or wireless communication means in case the state of consciousness indicates that the subject is unconscious and/or unresponsive. This may comprise announcing the analyte concentration (e.g. reading the glucose level aloud) determined in step 204 and/or information pertaining to the analyte concentration using the loudspeaker 108C. In some embodiments, the analyte concentration may be compared with a threshold and a message may be announced via the loudspeaker 108C depending on whether the analyte concentration is above or below the threshold. For example, if the glucose level is below a pre-defined threshold (e.g. below 70 mg/dL, in one example below 50 mg/dL), the device 100 may point out that the subject/user is hypoglycemic.

Additionally or alternatively, first aid instructions may be provided, e.g. announced via the loudspeaker 108C and/or displayed on the display 108B, to assist first responders, in particular inexperienced first responders such as random bystanders. The first aid instructions are based on the degree of absorption of the excitation radiation (e.g. the analyte concentration) at least in part. For example, different first aid instructions may be announced depending on whether the analyte concentration is above or below a threshold. For example, if the glucose level is below a pre-defined threshold (e.g. below 70 mg/dL, in one example below 50 mg/dL), the device 100 may point out that the subject/user is hypoglycemic and may instruct the first responders to give food and/or a sugary drink to the subject.

Step 212 may further comprise providing medical information pertaining to a general state of health of the subject, in particular to one or more medical conditions of the subject, via the communication unit 108. For example, the device 100 may announce via the loudspeaker 108C that the subject has diabetes and/or other medical conditions such as allergies.

In some embodiments, step 212 may also comprise automatically making an emergency call, e.g. via the wireless communication module 108D. This may further comprise providing monitoring information pertaining to the degree of absorption of said excitation radiation and/or medical information as part of the emergency call, e.g. an analyte concentration, a time trace of the analyte concentration and/or information pertaining to the analyte concentration, for example whether the analyte concentration is above or below a threshold (e.g. whether the subject is hypoglycemic or not).

In some embodiments, some or all of the actions taken in step 212 may be performed using an external device such as a smartphone of the subject. For example, the controller 34 may control the wireless communication module 108D to provide the respective information to the external device and to cause the external device to provide this information (e.g. to announce the information via a loudspeaker of the device) and/or to make the emergency call.

The method 200 may further comprise, in step 214, reducing a time delay between subsequent analyte measurements, e.g. by increasing a frequency of the analyte measurements (monitoring frequency). For example, the frequency of the analyte measurement may be increased from the first frequency used previously, e.g. in step 204 prior to determining that the subject is unconscious and/or unresponsive, to a second frequency that is higher than the first frequency. The first frequency may e.g. be between once every 5 minutes to once every hour, whereas the second frequency may e.g. be between once every second to once every minute. In this way, monitoring information with increased accuracy may be provided in case of emergency at the expense of an increased energy consumption of the device 100.

Subsequently, the method 200 may return to steps 202 and 204 to continue monitoring of the subject using the auxiliary sensors and/or the analyte measurements, e.g. with the increased second frequency for performing the analyte measurements in step 204. In steps 206 and 208, the device 100 may then determine again whether the subject is still unconscious and/or unresponsive and, if this is the case, may for example proceed to step 212 to provide updated monitoring information (e.g. the most recent analyte concentration or a rate of change of the analyte concentration) and/or updated first aid instructions.

While the present invention has been described in terms of specific embodiments, it is understood that variations and modifications will occur to those in the art, all of which are intended as aspects of the present invention. Accordingly, only such limitations as appear in the claims should be placed on the invention.

## Claims

1. A device (100) for monitoring an analyte in tissue of a human or animal subject, the device (100) comprising:

   a measurement body (16) having a contact surface (14) suitable to be brought in contact with a skin of the subject, said contact permitting heat and/or pressure waves generated by absorption of excitation radiation in the tissue to be transferred to said measurement body (16);
   an excitation radiation source (26) configured for irradiating excitation radiation at a plurality of wavelengths into the tissue to be absorbed by the analyte contained therein;
   a detection device (28, 30) for detecting a physical response of the measurement body (16) or of a component included therein to a heat and/or pressure wave received from the tissue upon absorption of the excitation radiation and for generating a response signal based on said detected physical response, said response signal being indicative of a degree of absorption of excitation radiation;
   a controller (34) to control the excitation radiation source (26) to irradiate excitation radiation into the tissue to be absorbed by the analyte contained therein and to control the detection device (28, 30) to detect said physical response and to generate a response signal indicative of the degree of absorption of said excitation radiation;
   a holding portion (104) for securing the device (100) to a body part (106), in particular a limb of the subject; and
   a communication unit (108) configured to provide monitoring information pertaining to the degree of absorption of said excitation radiation and/or a first aid instruction based on the degree of absorption of said excitation radiation by acoustic, optical, and/or wireless communication means;

   **characterized in that** the controller (34) is configured to:

determine a state of consciousness of the subject that characterizes whether the subject is conscious and/or responsive, wherein the state of consciousness is determined based on said response signal and/or based on a sensor signal from one or more auxiliary sensors (110, 112) configured to measure a parameter indicative of whether the subject is conscious and/or responsive; and

in response to the state of consciousness indicating that the subject is unconscious and/or unresponsive, provide monitoring information pertaining to the degree of absorption of said excitation radiation and/or a first aid instruction based on the degree of absorption of said excitation radiation via the communication unit (108) by one or more of said acoustic, optical, and/or wireless communication means.

2. The device (100) of claim 1, wherein said analyte is glucose, in particular wherein said tissue is the skin of the subject and said analyte is glucose present in an intersitital fluid thereof.

3. The device (100) of claim 1 or 2, wherein the controller (34) is configured to determine a concentration of the analyte in the tissue based on the response signal generated by the detection device and the monitoring information quantifies the determined concentration of the analyte and/or indicates whether the determined concentration is below or above one or more thresholds.

4. The device (100) of any one of the preceding claims, wherein the controller (34) is further configured to provide medical information pertaining to a general state of health of the subject, in particular to one or more medical conditions of the subject, via the communication unit (108) if the state of consciousness indicates that the subject is unconscious and/or unresponsive.

5. The device (100) of any one of the preceding claims, wherein the controller (34) is configured to determine the state of consciousness based on the sensor signal from the one or more auxiliary sensors (110, 112) and the one or more auxiliary sensors (110, 112) comprise one or more of:

a pulse sensor (110) configured to determine a pulse of the subject;
an oxygen saturation sensor (110) configured to determine an oxygen saturation level of blood of the subject;
a breathing sensor configured to determine a breathing rate of the subject and/or whether the subject is breathing;
a blood pressure sensor configured to determine a blood pressure of the subject;
an acceleration sensor (112) configured to determine an acceleration and/or a direction of gravity; and
a position sensor configured to determine a position.

6. The device (100) of claim 5, wherein the device (100) comprises some or all of the one or more auxiliary sensors (110, 112).

7. The device (100) of any one of the preceding claims, wherein the controller (34) is configured to determine a concentration of the analyte in the tissue based on the response signal generated by the detection device and to determine the state of consciousness based on the determined concentration.

8. The device (100) of any one of the preceding claims, wherein the communication unit (108) comprises a display (108B) for displaying the monitoring information, the first aid instruction and/or the medical information and/or a loudspeaker (108C) for acoustic playback of the monitoring information, the first aid instruction and/or the medical information.

9. The device (100) of any one of the preceding claims, wherein the communication unit (108) comprises a wireless communication module (108D) configured to make an emergency call and/or to communicate with an external device, in particular a portable computing device,
wherein the controller (34) is preferably configured to:

transmit the monitoring information, the first aid instruction and/or the medical information to the external device via the wireless communication module (108D); and
cause the external device to display the monitoring information, the first aid instruction and/or the medical information on a display of the external device, playback the monitoring information, the first aid instruction and/or the medical information via a loudspeaker of the external device, provide the monitoring information, the first aid instruction and/or the medical information by wireless communication means of the external device and/or make an emergency call.

10. The device (100) of any one of the preceding claims, wherein the controller (34) is configured to change from a first means (108B) for providing the monitoring information to a second means (108C, 108D) for providing the monitoring information based on the state of consciousness, the response signal and/or the sensor signal from the one or more auxiliary sensors (110, 112).

**11.** The device (100) of any one of the preceding claims, wherein the controller (34) is configured to repeatedly perform analyte measurements by controlling the excitation radiation source (26) to irradiate excitation radiation into the tissue to be absorbed by the analyte contained therein and to control the detection device (28, 30) to detect said physical response and to generate a response signal indicative of the degree of absorption of said excitation radiation,

> wherein the controller (34) is preferably further configured to adjust a delay time between subsequent analyte measurements based on the state of consciousness, the response signal and/or the sensor signal from the one or more auxiliary sensors (110, 112), and/or
> wherein said analyte is glucose and the controller (34) is configured to record a time trace of a concentration of glucose in said tissue and to determine a point in time of a most recent food intake by the subject based on said time trace.

**Patentansprüche**

**1.** Vorrichtung (100) zum Überwachen eines Analyten in Gewebe eines menschlichen oder tierischen Subjekts, wobei die Vorrichtung (100) Folgendes umfasst:

> einen Messkörper (16) mit einer Kontaktfläche (14), die geeignet ist, mit einer Haut des Subjekts in Kontakt gebracht zu werden, wobei der Kontakt ermöglicht, dass Wärme- und/oder Druckwellen, die durch Absorption von Anregungsstrahlung in dem Gewebe erzeugt werden, auf den Messkörper (16) übertragen werden;
> eine Anregungsstrahlungsquelle (26), die konfiguriert ist, um Anregungsstrahlung bei einer Vielzahl von Wellenlängen in das Gewebe einzustrahlen, um von dem darin enthaltenen Analyten absorbiert zu werden;
> eine Detektionsvorrichtung (28, 30) zum Detektieren einer physikalischen Reaktion des Messkörpers (16) oder einer darin enthaltenen Komponente auf eine Wärme- und/oder Druckwelle, die von dem Gewebe bei Absorption der Anregungsstrahlung empfangen wird, und zum Erzeugen eines Reaktionssignals basierend auf der detektierten physikalischen Reaktion, wobei das Reaktionssignal einen Grad der Absorption von Anregungsstrahlung anzeigt;
> eine Steuerung (34), um die Anregungsstrahlungsquelle (26) zu steuern, Anregungsstrahlung in das Gewebe einzustrahlen, um von dem darin enthaltenen Analyten absorbiert zu werden, und um die Detektionsvorrichtung (28,

> 30) zu steuern, die physikalische Reaktion zu detektieren und ein Reaktionssignal zu erzeugen, das den Grad der Absorption der Anregungsstrahlung anzeigt;
> einen Halteteil (104) zum Befestigen der Vorrichtung (100) an einem Körperteil (106), insbesondere einer Gliedmaße des Subjekts; und
> eine Kommunikationseinheit (108), die konfiguriert ist, um Überwachungsinformation, die den Grad der Absorption der Anregungsstrahlung betrifft, und/oder eine Erste-Hilfe-Anweisung basierend auf dem Grad der Absorption der Anregungsstrahlung durch akustische, optische und/oder drahtlose Kommunikationsmittel bereitzustellen;

> **dadurch gekennzeichnet, dass** die Steuerung (34) konfiguriert ist, um:

> einen Bewusstseinszustand des Subjekts zu bestimmen, der charakterisiert, ob das Subjekt bei Bewusstsein und/oder reaktionsfähig ist, wobei der Bewusstseinszustand basierend auf dem Reaktionssignal und/oder basierend auf einem Sensorsignal von einem oder mehreren Hilfssensoren (110, 112) bestimmt wird, die konfiguriert sind, um einen Parameter zu messen, der anzeigt, ob das Subjekt bei Bewusstsein und/oder reaktionsfähig ist; und
> als Reaktion darauf, dass der Bewusstseinszustand anzeigt, dass das Subjekt nicht bei Bewusstsein und/oder nicht reaktionsfähig ist, Überwachungsinformation, die den Grad der Absorption der Anregungsstrahlung betrifft, und/oder eine Erste-Hilfe-Anweisung basierend auf dem Grad der Absorption der Anregungsstrahlung über die Kommunikationseinheit (108) durch eines oder mehrere der akustischen, optischen und/oder drahtlosen Kommunikationsmittel bereitzustellen.

**2.** Vorrichtung (100) nach Anspruch 1, wobei der Analyt Glukose ist, insbesondere wobei das Gewebe die Haut des Subjekts ist und der Analyt Glukose ist, die in einer interstitiellen Flüssigkeit davon vorhanden ist.

**3.** Vorrichtung (100) nach Anspruch 1 oder 2, wobei die Steuerung (34) konfiguriert ist, um eine Konzentration des Analyten in dem Gewebe basierend auf dem von der Detektionsvorrichtung erzeugten Reaktionssignal zu bestimmen, und die Überwachungsinformation die bestimmte Konzentration des Analyten quantifiziert und/oder anzeigt, ob die bestimmte Konzentration unter oder über einem oder mehreren Schwellenwerten liegt.

**4.** Vorrichtung (100) nach einem der vorhergehenden Ansprüche, wobei die Steuerung (34) ferner konfiguriert ist, um medizinische Information, die einen allgemeinen Gesundheitszustand des Subjekts, insbesondere eine oder mehrere Krankheiten des Subjekts, betrifft, über die Kommunikationseinheit (108) bereitzustellen, wenn der Bewusstseinszustand anzeigt, dass das Subjekt nicht bei Bewusstsein und/oder nicht reaktionsfähig ist.

5. Vorrichtung (100) nach einem der vorhergehenden Ansprüche, wobei die Steuerung (34) konfiguriert ist, um den Bewusstseinszustand basierend auf dem Sensorsignal von dem einen oder den mehreren Hilfssensoren (110, 112) zu bestimmen, und der eine oder die mehreren Hilfssensoren (110, 112) einen oder mehreren der Folgenden umfassen:

einen Pulssensor (110), der konfiguriert ist, um einen Puls des Subjekts zu bestimmen;
einen Sauerstoffsättigungssensor (110), der konfiguriert ist, um ein Sauerstoffsättigungslevel von Blut des Subjekts zu bestimmen;
einen Atmungssensor, der konfiguriert ist, um eine Atmungsrate des Subjekts zu bestimmen und/oder ob das Subjekt atmet;
einen Blutdrucksensor, der konfiguriert ist, um einen Blutdruck des Subjekts zu bestimmen;
einen Beschleunigungssensor (112), der konfiguriert ist, um eine Beschleunigung und/oder eine Richtung der Schwerkraft zu bestimmen; und
einen Positionssensor, der konfiguriert ist, um eine Position zu bestimmen.

6. Vorrichtung (100) nach Anspruch 5, wobei die Vorrichtung (100) einige oder alle des einen oder der mehreren Hilfssensoren (110, 112) umfasst.

7. Vorrichtung (100) nach einem der vorhergehenden Ansprüche, wobei die Steuerung (34) konfiguriert ist, um eine Konzentration des Analyten in dem Gewebe basierend auf dem von der Detektionsvorrichtung erzeugten Reaktionssignal zu bestimmen und den Bewusstseinszustand basierend auf der bestimmten Konzentration zu bestimmen.

8. Vorrichtung (100) nach einem der vorhergehenden Ansprüche, wobei die Kommunikationseinheit (108) eine Anzeige (108B) zum Anzeigen der Überwachungsinformation, der Erste-Hilfe-Anweisung und/oder der medizinischen Information und/oder einen Lautsprecher (108C) zur akustischen Wiedergabe der Überwachungsinformation, der Erste-Hilfe-Anweisung und/oder der medizinischen Information umfasst.

9. Vorrichtung (100) nach einem der vorhergehenden Ansprüche, wobei die Kommunikationseinheit (108) ein drahtloses Kommunikationsmodul (108D) umfasst, das konfiguriert ist, um einen Notruf zu tätigen und/oder mit einer externen Vorrichtung, insbesondere einer tragbaren Rechnervorrichtung, zu kommunizieren,
wobei die Steuerung (34) vorzugsweise konfiguriert ist, um:

die Überwachungsinformation, die Erste-Hilfe-Anweisung und/oder die medizinischen Information über das drahtlose Kommunikationsmodul (108D) an die externe Vorrichtung zu übertragen; und
die externe Vorrichtung zu veranlassen, die Überwachungsinformation, die Erste-Hilfe-Anweisung und/oder die medizinischen Information auf einer Anzeige der externen Vorrichtung anzuzeigen, die Überwachungsinformation, die Erste-Hilfe-Anweisung und/oder die medizinischen Information über einen Lautsprecher der externen Vorrichtung wiederzugeben, die Überwachungsinformation, die Erste-Hilfe-Anweisung und/oder die medizinischen Information durch drahtlose Kommunikationsmittel der externen Vorrichtung bereitzustellen und/oder einen Notruf zu tätigen.

10. Vorrichtung (100) nach einem der vorhergehenden Ansprüche, wobei die Steuerung (34) konfiguriert ist, um von einem ersten Mittel (108B) zum Bereitstellen der Überwachungsinformation zu einem zweiten Mittel (108C, 108D) zum Bereitstellen der Überwachungsinformation basierend auf dem Bewusstseinszustand, dem Reaktionssignal und/oder dem Sensorsignal von dem einen oder den mehreren Hilfssensoren (110, 112) zu wechseln.

11. Vorrichtung (100) nach einem der vorhergehenden Ansprüche, wobei die Steuerung (34) konfiguriert ist, um wiederholt Analytmessungen durch Steuern der Anregungsstrahlungsquelle (26) durchzuführen, um Anregungsstrahlung in das Gewebe einzustrahlen, um von dem darin enthaltenen Analyten absorbiert zu werden, und um die Detektionsvorrichtung (28, 30) zu steuern, die physikalische Reaktion zu detektieren und ein Reaktionssignal zu erzeugen, das den Grad der Absorption der Anregungsstrahlung anzeigt,

wobei die Steuerung (34) vorzugsweise ferner konfiguriert ist, um eine Verzögerungszeit zwischen nachfolgenden Analytmessungen basierend auf dem Bewusstseinszustand, dem Reaktionssignal und/oder dem Sensorsignal von dem einen oder den mehreren Hilfssensoren (110, 112) anzupassen, und/oder
wobei der Analyt Glukose ist und die Steuerung (34) konfiguriert ist, um eine Zeitspur einer Konzentration von Glukose in dem Gewebe aufzuzeichnen und einen Zeitpunkt einer jüngsten Nahrungsaufnahme durch das Subjekt basierend auf der Zeitspur zu bestimmen.

## Revendications

1. Dispositif (100) de surveillance d'un analyte dans le tissu d'un sujet humain ou animal, le dispositif (100) comprenant :

   un corps de mesure (16) ayant une surface de contact (14) adaptée pour être mise en contact avec une peau du sujet, ledit contact permettant à des ondes de chaleur et/ou de pression générées par absorption de rayonnement d'excitation dans le tissu d'être transférées vers ledit corps de mesure (16) ;
   une source de rayonnement d'excitation (26) configurée pour irradier un rayonnement d'excitation à une pluralité de longueurs d'onde dans le tissu pour qu'il soit absorbé par l'analyte contenu à l'intérieur de celui-ci ;
   un dispositif de détection (28, 30) pour détecter une réponse physique du corps de mesure (16) ou d'un composant inclus dans celui-ci à une onde de chaleur et/ou de pression reçue du tissu lors de l'absorption du rayonnement d'excitation et pour générer un signal de réponse sur la base de ladite réponse physique détectée, ledit signal de réponse indiquant un degré d'absorption de rayonnement d'excitation ;
   un dispositif de commande (34) pour commander la source de rayonnement d'excitation (26) afin d'irradier le rayonnement d'excitation dans le tissu pour qu'il soit absorbé par l'analyte contenu à l'intérieur de celui-ci et pour commander le dispositif de détection (28, 30) afin de détecter ladite réponse physique et de générer un signal de réponse indiquant le degré d'absorption dudit rayonnement d'excitation ;
   une portion de maintien (104) pour fixer le dispositif (100) à une partie de corps (106), en particulier un membre du sujet ; et
   une unité de communication (108) configurée pour fournir des informations de surveillance concernant le degré d'absorption dudit rayonnement d'excitation et/ou une instruction de premier secours sur la base du degré d'absorption dudit rayonnement d'excitation par des moyens de communication acoustiques, optiques et/ou sans fil ;
   **caractérisé en ce que** le dispositif de commande (34) est configuré pour :

      déterminer un état de conscience du sujet qui caractérise si le sujet est conscient et/ou réactif, dans lequel l'état de conscience est déterminé sur la base dudit signal de réponse et/ou sur la base d'un signal de capteur provenant d'un ou plusieurs capteurs auxiliaires (110, 112) configurés pour mesurer un paramètre indiquant si le sujet est conscient et/ou réactif ; et
   en réponse au fait que l'état de conscience indique que le sujet est inconscient et/ou non réactif, fournir des informations de surveillance concernant le degré d'absorption dudit rayonnement d'excitation et/ou une instruction de premier secours sur la base du degré d'absorption dudit rayonnement d'excitation via l'unité de communication (108) par un ou plusieurs desdits moyens de communication acoustiques, optiques et/ou sans fil.

2. Dispositif (100) selon la revendication 1, dans lequel ledit analyte est du glucose, en particulier dans lequel ledit tissu est la peau du sujet et ledit analyte est du glucose présent dans un fluide interstitiel de celui-ci.

3. Dispositif (100) selon la revendication 1 ou 2, dans lequel le dispositif de commande (34) est configuré pour déterminer une concentration de l'analyte dans le tissu sur la base du signal de réponse généré par le dispositif de détection et les informations de surveillance quantifient la concentration déterminée de l'analyte et/ou indiquent si la concentration déterminée est en dessous ou au-dessus d'un ou plusieurs seuils.

4. Dispositif (100) selon l'une quelconque des revendications précédentes, dans lequel le dispositif de commande (34) est en outre configuré pour fournir des informations médicales concernant un état de santé général du sujet, en particulier une ou plusieurs conditions médicales du sujet, via l'unité de communication (108) si l'état de conscience indique que le sujet est inconscient et/ou non réactif.

5. Dispositif (100) selon l'une quelconque des revendications précédentes, dans lequel le dispositif de commande (34) est configuré pour déterminer l'état de conscience sur la base du signal de capteur provenant des un ou plusieurs capteurs auxiliaires (110, 112) et les un ou plusieurs capteurs auxiliaires (110, 112) comprennent un ou plusieurs parmi :

   un capteur de pouls (110) configuré pour déterminer un pouls du sujet ;
   un capteur de saturation d'oxygène (110) configuré pour déterminer un niveau de saturation d'oxygène du sang du sujet ;
   un capteur de respiration configuré pour déterminer un rythme respiratoire du sujet et/ou si le sujet respire ;

un capteur de tension artérielle configuré pour déterminer une tension artérielle du sujet ;

un capteur d'accélération (112) configuré pour déterminer une accélération et/ou une direction de gravité ; et

un capteur de position configuré pour déterminer une position.

6. Dispositif (100) selon la revendication 5, dans lequel le dispositif (100) comprend certains ou la totalité des un ou plusieurs capteurs auxiliaires (110, 112).

7. Dispositif (100) selon l'une quelconque des revendications précédentes, dans lequel le dispositif de commande (34) est configuré pour déterminer une concentration de l'analyte dans le tissu sur la base du signal de réponse généré par le dispositif de détection et pour déterminer l'état de conscience sur la base de la concentration déterminée.

8. Dispositif (100) selon l'une quelconque des revendications précédentes, dans lequel l'unité de communication (108) comprend un affichage (108B) pour afficher les informations de surveillance, l'instruction de premier secours et/ou les informations médicales et/ou un haut-parleur (108C) pour la lecture acoustique des informations de surveillance, de l'instruction de premier secours et/ou des informations médicales.

9. Dispositif (100) selon l'une quelconque des revendications précédentes, dans lequel l'unité de communication (108) comprend un module de communication sans fil (108D) configuré pour effectuer un appel d'urgence et/ou pour communiquer avec un dispositif externe, en particulier un dispositif informatique portable,

dans lequel le dispositif de commande (34) est de préférence configuré pour :

transmettre les informations de surveillance, l'instruction de premier secours et/ou les informations médicales au dispositif externe via le module de communication sans fil (108D) ; et amener le dispositif externe à afficher les informations de surveillance, l'instruction de premier secours et/ou les informations médicales sur un affichage du dispositif externe, lire les informations de surveillance, l'instruction de premier secours et/ou les informations médicales via un haut-parleur du dispositif externe, fournir les informations de surveillance, l'instruction de premier secours et/ou les informations médicales par des moyens de communication sans fil du dispositif externe et/ou effectuer un appel d'urgence.

10. Dispositif (100) selon l'une quelconque des revendications précédentes, dans lequel le dispositif de commande (34) est configuré pour passer d'un premier moyen (108B) de fourniture des informations de surveillance à un deuxième moyen (108C, 108D) de fourniture des informations de surveillance sur la base de l'état de conscience, du signal de réponse et/ ou du signal de capteur provenant des un ou plusieurs capteurs auxiliaires (110, 112).

11. Dispositif (100) selon l'une quelconque des revendications précédentes, dans lequel le dispositif de commande (34) est configuré pour réaliser de manière répétée des mesures d'analyte en commandant la source de rayonnement d'excitation (26) pour irradier le rayonnement d'excitation dans le tissu afin qu'il soit absorbé par l'analyte contenu à l'intérieur de celui-ci et pour commander le dispositif de détection (28, 30) afin de détecter ladite réponse physique et de générer un signal de réponse indiquant le degré d'absorption dudit rayonnement d'excitation,

dans lequel le dispositif de commande (34) est de préférence configuré en outre pour ajuster un temps de retard entre des mesures d'analyte ultérieures sur la base de l'état de conscience, du signal de réponse et/ou du signal de capteur provenant des un ou plusieurs capteurs auxiliaires (110, 112), et /ou

dans lequel ledit analyte est le glucose et le dispositif de commande (34) est configuré pour enregistrer une trace temporelle d'une concentration de glucose dans ledit tissu et pour déterminer un moment d'une prise alimentaire la plus récente par le sujet sur la base de ladite trace temporelle.

**Fig. 1**

IR spectra of glucose at different concentrations

**Fig. 2**

**Fig. 3**

Clarke's Error Grid Analysis-raw Amplitude

1558 data points

20 subjects
of these
17 healthy,
2 type 1
diabetics,
1 type 2
diabetic

zone A: 95.4%
zone B: 4.0%
zone C: 0%
zone D: 0.6%
zone E: 0%

**Fig. 4**

**Fig. 5**

**Fig. 6**

Fig. 7

Fig. 8

200

determine sensor signals
from auxiliary sensors

202

irradiate tissue, detect
physical response and
generate response signal

204

determine
state of consciousness

206

no ← subject
unconscious?

208

yes

change means for
providing information

210

provide monitoring and
medical information and
first aid instructions

212

increase monitoring
frequency

214

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015193310 A1
- WO 2017097824 A1
- WO 2020094265 A1
- WO 2019110597 A2
- CN 108369183 A
- WO 2021233559 A1
- WO 201709782 A1